# EUROPEAN PATENT APPLICATION

(11) **EP 4 299 575 A1**
(43) Date of publication of application: **03.01.2024**
(21) Application number: 21927662.3
(22) Date of filing: 06.12.2021
(51) Int. Cl.: C07D 471/04, C07D 487/04, A61K 31/437, A61K 31/5025, A61K 31/519, A61P 35/00, A61P 37/00

(54) **BTK AND/OR BTK C481S SMALL MOLECULE INHIBITOR AND USE THEREOF**

(30) Priority: 26.02.2021 CN 202110216191
(71) Applicant: Arromax Pharmatech Co., Ltd., Suzhou, Jiangsu 215100 (CN)
(72) Inventor: XIE, Lingzhi, Jiangsu 215100 (CN); QIAO, Dandan, Jiangsu 215100 (CN); DEBASIS, Das, Jiangsu 215100 (CN); HONG, Jian, Jiangsu 215100 (CN)
(74) Representative: RGTH
(86) International application number: PCT/CN2021/135698
(87) International publication number: WO 2022/179238

(57) **Abstract**

The present invention provides a new compound having a structure of Formula I, or an enol compound, a pharmaceutically acceptable salt, a deuterated derivative, a hydrate or solvate, an active metabolite, a polymorph, an ester, an optical isomer or a prodrug thereof, a pharmaceutical composition comprising the compound having a structure of Formula I, and use thereof in the preparation of a drug that is a Bruton's tyrosine kinase (BTK) inhibitor or has selectivity for a **BTK** mutant (C481S) to prevent or treat immune-related diseases, autoimmune diseases or cancers.

## Description

### FIELD OF THE INVENTION

The present invention relates to a new compound, and more particularly to a compound of Formula I as a kinase inhibitor, or a pharmaceutically acceptable salt, a solvate, an active metabolite, a polymorph, an ester, an optical isomer, or a prodrug, and/or a pharmaceutical composition thereof. In particular, the present invention relates to inhibitors against Bruton's tyrosine kinase (BTK) and/or a mutated BTK mutant (C481S).

### DESCRIPTION OF THE RELATED ART

Bruton's tyrosine kinase (**BTK**) is essential for **B** cell receptor (**BCR**)-mediated signals and response to maintain **B** cell bank (Hunter, cell, 1987 50, 823-829) and for the regulation of early **B** cell development and activation of mature **B** cells. The signal control a series of effect reactions through **BCR**, including the activation, proliferation and differentiation of mature antibody-producing cells. **BTK** is also expressed in other hematopoietic cells, such as monocytes, macrophages and mast cells, and responsible for the regulation of some immune responses, such as **TNF** production stimulated by **Fc** receptors. Therefore, the **TNF**-mediated inflammation can be regulated by small molecular **BTK** inhibitors. Bruton's tyrosine kinase (**BTK**) plays an important role in **B** cell receptor **(BCR)** and **Fcy** receptor **(FcyR)** signaling pathways, and regulates the activation, proliferation, differentiation and survival of **B** cells. Abnormal **BTK** function can be observed in **B** cell derived malignant tumors, including mantle cell lymphoma (**MCL**), chronic lymphoblastic leukemia (**CLL**), acute lymphoblastic leukemia (**ALL**), non-Hodgkin's lymphoma (**NHL**), Waldenström's macroglobulinemia (**WM**) and multiple myeloma (**MM**) (Nature. 2010; 463(7277): 88-92). Therefore, **BTK** is considered as an important therapeutic target for treating B-cell malignant tumors and autoimmune diseases *(*Expert Opinion on Investigational Drugs. 2017; 26(8):909-915). I*brutinib* is the first irreversible **BTK** inhibitor *(*Future Oncology. 2014; 10 (6):957-967) approved by **FDA** for the treatment of mantle cell lymphoma (**MCL**), chronic lymphoblastic leukemia (**CLL**) and Waldenström's macroglobulinemia (**WM**) *(*New England Journal of Medicine. 2013; 369 (6): 507-516). Ibrutinib can effectively inhibit the proliferation of some types of **DLBCL.** The mechanism of action of Ibrutinib is to covalently cross-link with the sulfhydryl structure at position 481 of **BTK** protein in cells, thus depriving **BTK** of its function of phosphorylating the downstream signal protein and exerting an anti-proliferation effect. Although Ibrutinib has a significant clinical effect on **B** cell derived malignant tumor, drug resistance is developed in primary and secondary patients, the prognosis is poor and the options for treatment is limited. In addition, Ibrutinib has a strong inhibitory activity against **BTK** kinase, and the drug itself has a high off-target effect, and causes many side effects, such as diarrhea and rash, leads to the function loss of natural killer cells and causes coagulation disorders. Most patients with **CLL** who are resistant to irreversible **BTK** inhibitor such as Ibrutinib have **BTK-C481S** mutation. It is reported that 80% of patients with reoccurring **CLL** have **C481S** mutation (Maddocks KJ, et al. JAMA Oncol. 2015; 1:80-87). Another research group reported that in the fourth year, about 20% of patients receiving Ibrutinib are clinically progressive, and 85% of patients had **C481S** mutation (Journal of Clinical Oncology Vol 35, number 13, 2017, page 1437). In addition, these mutations were detected within 9 months on average before the recurrence. It is reported that **C481S** mutation of **BTK** kinase can significantly reduce the sensitivity to Ibrutinib. **BTK** has become a new molecular target for the treatment of **B** cell lymphoma, leukemia and autoimmune diseases (Shinohara et al, Cell 132(2008) pp794-806; Gilfillan et al, Immunological Reviews 288 (2009) pp 149- 169; and Davis et al, Nature, 463 (2010) pp 88-94). Targeting **BTK** with small molecule inhibitor may have advantages over biotherapy with **RA**, for example, in the regulation of **B** cell response and/or activation, while desirable immunity is maintained (Franks, S.E., et al., Current Opinion in Immunology (2016), 43: 39-45).

Inhibition of **BTK** with small molecule inhibitor has the potential to immune disorders, cancers, cardiovascular diseases, viral infections, inflammation, metabolic/endocrine disorders and nervous system diseases. Therefore, it is still a focus and need in future research to find new small molecular compound with **BTK** inhibitory activity and inhibitors against **BTK (C481S)** mutation.

### SUMMARY OF THE INVENTION

After in-depth study, the inventors found that the compound represented by Formula I has inhibitory activity against **BTK,** particularly for mutated **BTK C481S** mutant. The compound of Formula **I** or a stereoisomer, a geometric isomer, a tautomer, a N-oxide, a hydrate, a solvate, a metabolites or a pharmaceutically acceptable salt or prodrug thereof can be used to treat cancers, allergic diseases, autoimmune diseases, inflammatory diseases, and others, particularly **B** cell lymphoma and leukemia patients.

The present invention relates to a novel effective and selective **BTK** inhibitor, which is reasonably designed to inhibit WT **BTK** and/or **BTK** mutation (**C481S**).

Therefore, the present invention provides a compound of Formula I, and an enol ester, a pharmaceutically acceptable salt, a solvate, and a composition thereof, where R, G, W¹, W², W³, X, and Y are as defined herein,

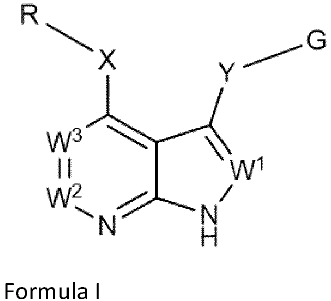

In Formula I,
R is independently selected from a substituted or unsubstituted alkyl, an alkenyl group, an alcohol in which the alkyl group is unsubstituted or substituted, an amine in which the alkyl group is unsubstituted or substituted, a substituted or unsubstituted four to seven-membered carbocyclic ring, and a substituted or unsubstituted four to seven-membered carbocyclic ring having a heteroatom including, but not limited to, N, S, and O.

Preferably, R is independently selected from a substituted or unsubstituted C₁-C₆ alkyl or C₂-C₄ alkenyl group, a C₁-C₆ alcohol in which the alkyl group is unsubstituted or substituted, a C₁-C₆alkyl amine in which the alkyl group is unsubstituted or substituted, a substituted or unsubstituted four to seven-membered carbocyclic ring, and a substituted or unsubstituted four to seven-membered carbocyclic ring having a heteroatom including, but not limited to, N, S, and O.

The substituent is selected from amino, an ester group, carboxyl, hydroxyl, acylamino(including N- and C-), sulfonyl, sulfonamido, C₁-C₄ alkyl, C₁-C₄ alkylamine, C₁-C₄ alkyl alcohol, and C₁-C₄ alkyl ether.

G is independently selected from a five- or six-membered aryl, substituted aryl, heteroaryl, substituted heteroaryl, biphenylyl, carbocyclic ring, carbocyclic ring containing a heteroatom, and unsaturated carbocyclic ring.

W¹, W², and W³ are independently selected from CH or N that satisfies the valence bond theory.

X is independently selected from O, N, and NH.

Y is independently selected from, but is not limited to, 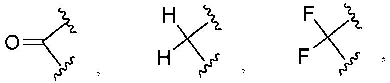 where when Y is absent, a chemical bond exists between the G unit and the heteroaryl ring. The present invention also provides a method of using a mixture comprising the compound of general Formula I or a pharmaceutically acceptable salt thereof and a pharmaceutically acceptable diluent or carrier.

Also provided herein is a method for inhibiting cell proliferation in vitro or in vivo, which comprises contacting a cell with an effective amount of the compound of Formula I or a pharmaceutically acceptable salt or a solvate thereof, or a pharmaceutical composition thereof as defined herein.

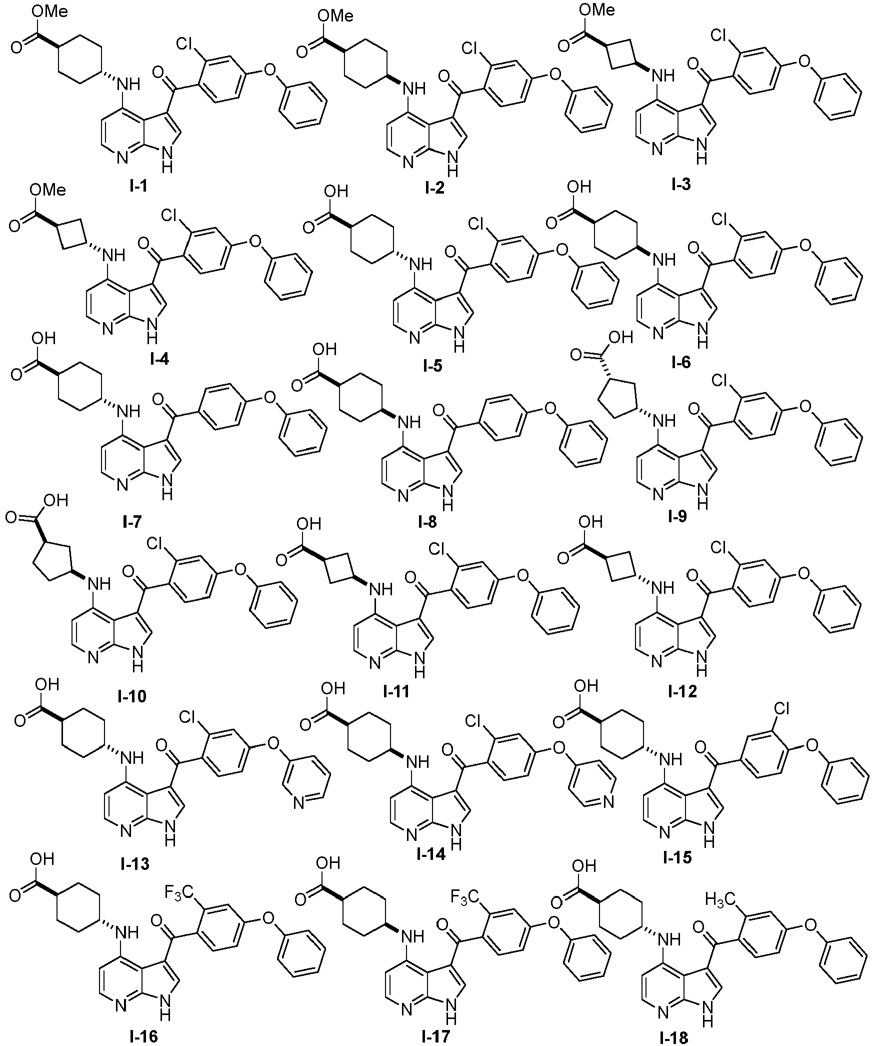

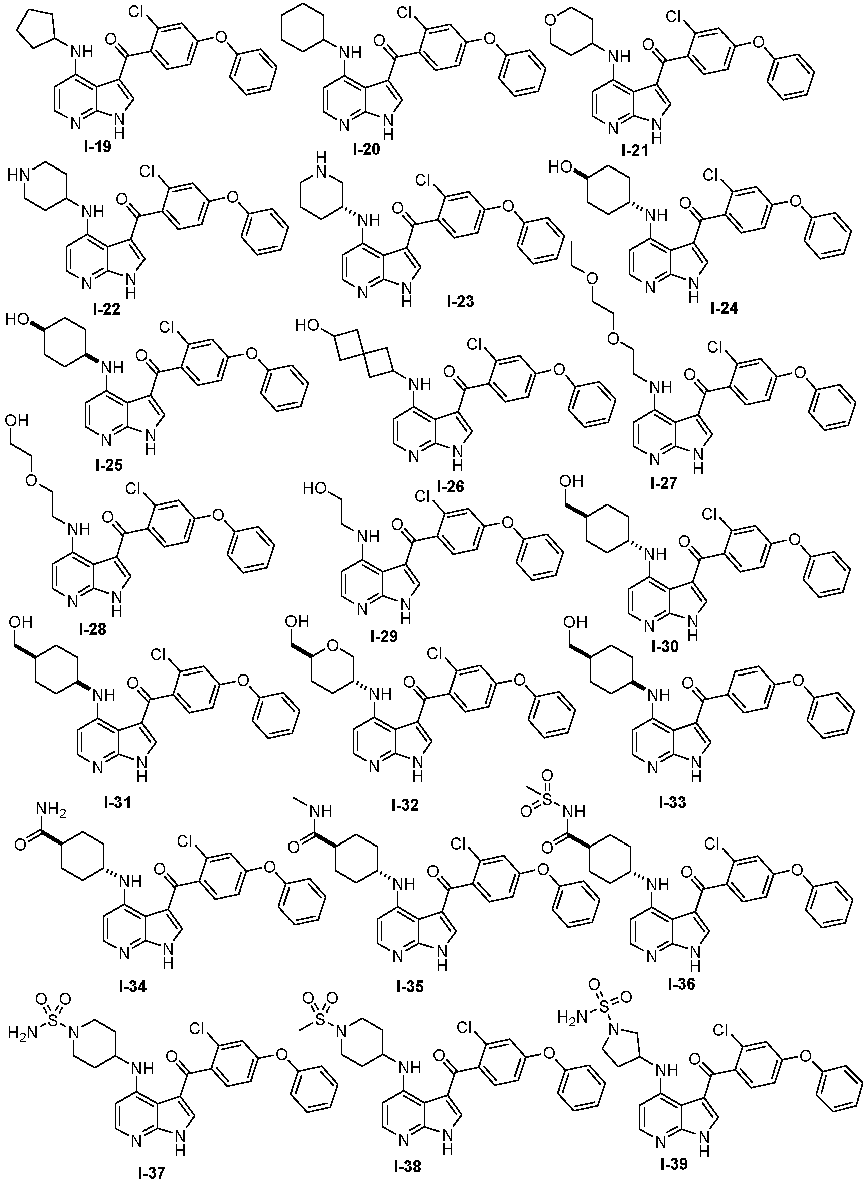

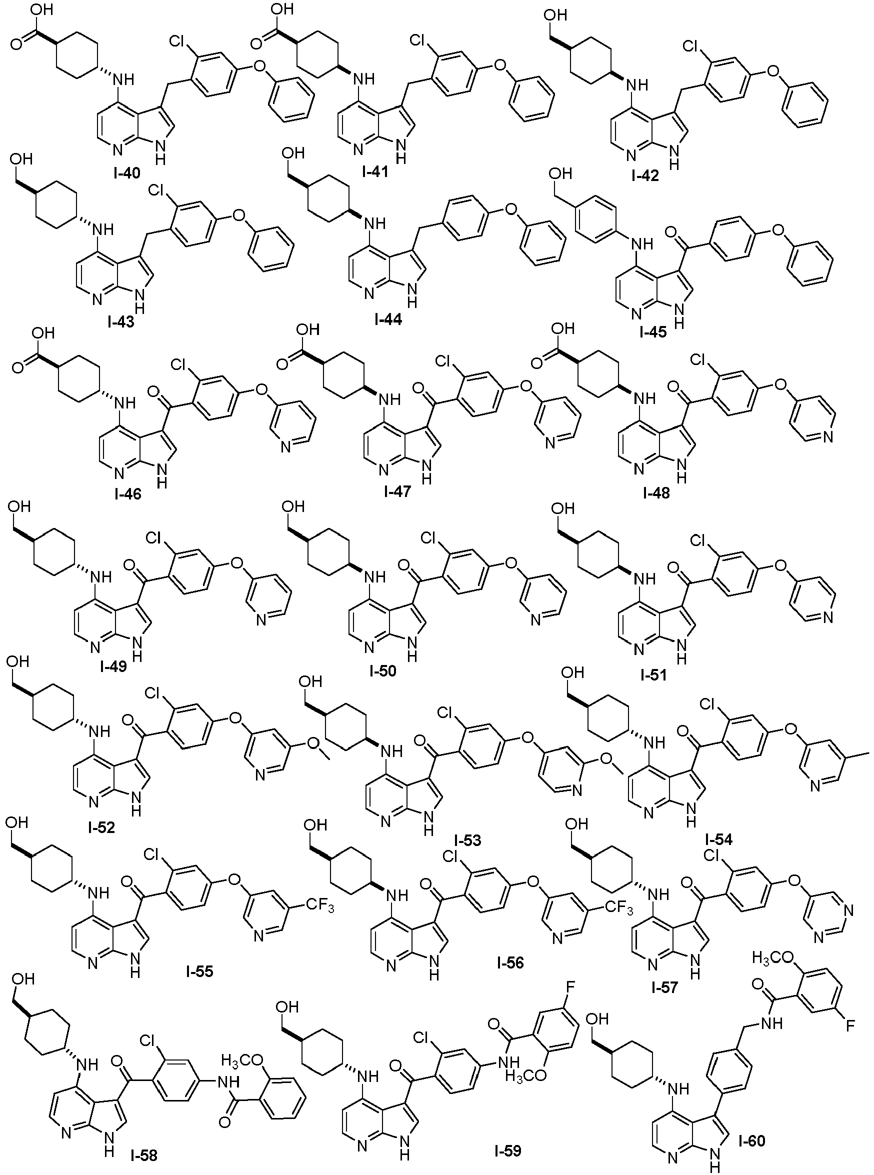

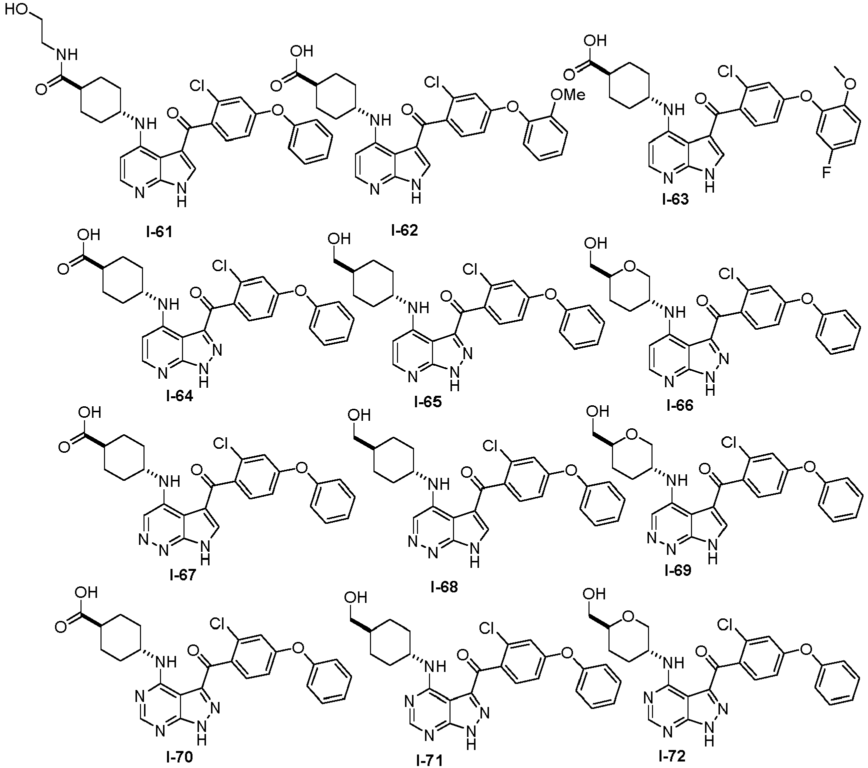

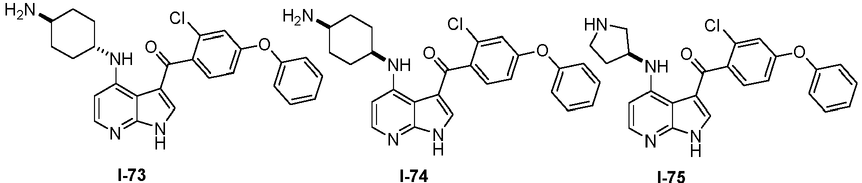

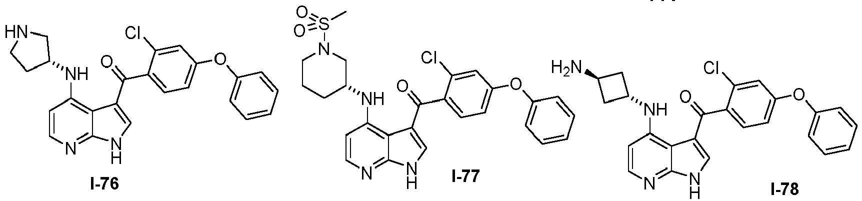

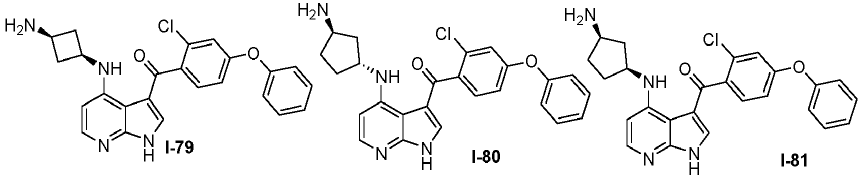

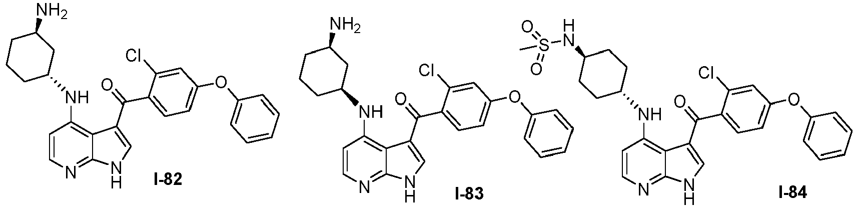

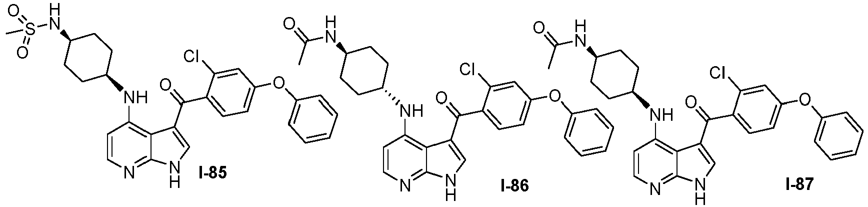

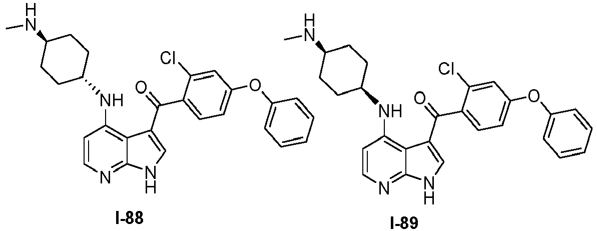

An object of the present invention is to provide a small molecular compound with **BTK** inhibitory activity. The compound of the present invention can be used for treating patients who are resistant/refractory to the first-generation **BTK** inhibitor (such as Ibrutinib and Acatinib), particularly patients with **BTK C481S** mutation. The present invention provides a compound of Formula I, or a pharmaceutically acceptable salt, or a stereoisomer or tautomer thereof, and a pharmaceutically acceptable salt, hydrate or solvate thereof; and a pharmaceutically acceptable carrier, or a stereoisomer or tautomer, and a pharmaceutically acceptable salt, hydrate or solvate thereof. According to the second aspect of the present invention, the invention provides a pharmaceutical composition and its use in the preparation of drugs for preventing and/or treating abnormal activation of **BTK** and diseases related to abnormal activation of **BTK** mutation **C481S.**

The compound of the present invention can be used for treating patients with diseases such as autoimmune diseases, inflammatory diseases or cancers, including but not limited to **B-**cell-derived malignant tumors (**MCL**), chronic lymphocytic leukemia (**CLL**), acute lymphocytic leukemia (**ALL**), non-Hodgkin's lymphoma (**NHL**), Waldenström's macroglobulinaemia (**WM**) and multiple myeloma (**MM**).

### DETAILED DESCRIPTION OF THE PREFERRED EMBODIMENTS

The compound of the present invention is represented by Formula **I**, and examples, sub-examples, and the structures of Formula **Ia,** Formula **Ib,** Formula **Ic,** Formula **Id,** Formula **le,** Formula **If** are disclosed herein. The abbreviations used herein have conventional meanings in chemistry and biology. As used herein, unless otherwise specified, the following definitions and terms shall apply.

The "R" and "S" describing isomers are descriptors of the stereochemical configuration of asymmetrically substituted carbon atoms. The naming of asymmetrically substituted carbon atoms as "R" or "S" is accomplished by applying the **Cahn-Ingold Prelog** priority rules, which are also known to those skilled in the art and described in the naming rules of organic chemistry of the International Union of Pure and Applied Chemistry **(IUPAC).** Section E, Stereochemistry.

As used herein, the term Cᵢ₋ⱼ means that the moiety has i-j carbon atoms. For example, C₁₋₁₀ alkyl means that the alkyl unit has any number of carbon atoms between 1 and 10.

As used herein, "alkyl" refers to a fully saturated straight, branched or cyclic hydrocarbon chain or a combination thereof. The alkyl group can be saturated, monounsaturated or polyunsaturated, and include divalent or multivalent groups, with a specified number of carbon atoms (i.e., C₁-C₁₀ refers to one to ten carbon atoms). In some embodiments, the alkyl group contains 1-6 carbon atoms. In some embodiments, the alkyl group contains 1-4 carbon atoms. In some embodiments, the alkyl group contains 1-3 carbon atoms. In other embodiments, the alkyl group contains 2-3 carbon atoms, and in other embodiments, the alkyl group contains 1-2 carbon atoms. In some embodiments, the term "alkyl" refers to a cycloalkyl, also known as a carbocyclic ring. Examples of saturated hydrocarbon groups include, but are not limited to, for example, methyl, ethyl, n-propyl, isopropyl, n-butyl, tert-butyl, isobutyl, sec-butyl, n-pentyl, n-hexyl, n-heptyl, n-octyl, cyclohexyl, cyclohexylmethyl, and the like. Unsaturated hydrocarbon groups are hydrocarbon groups with one or more double bonds or triple bonds. Example of unsaturated hydrocarbon groups include, but are not limited to, ethenyl, 2- propenyl, crotyl, 2-isopentenyl, 2-(butadienyl), 2,4-pentadienyl, 3-(1,4-pentadienyl), ethynyl, 1- and 3-propynyl, 3-butenyl and higher homologues and isomers. The alkyl is optionally substituted with one or more halo. For example, the term "fluoroalkyl" refers to an alkyl group as defined above, in which one or more hydrogen atoms are replaced by fluorine atoms.

The term "alkoxy" refers to a straight-chain or branched alkoxy with an indicated number of carbon atoms. For example, C₁₋₆ alkoxy includes methoxy, ethoxy, propoxy, isopropoxy, and the like.

The term "hydrocarbylene" itself or as a moiety of another substituent refers to a divalent group derived from a hydrocarbyl group, such as -CH₂CH₂CH₂CH₂-, -CH₂CH=CH₂-, -CH₂C≡CCH₂-, -CH₂CH₂CH(CH₂CH₂CH₃)CH₂-, without limitation. The hydrocarbyl (or hydrocarbylene) generally has 1 to 24 carbon atoms, and is preferably a group having 10 or less carbon atoms in the present invention.

The term "alkynyl" refers to a carbon chain containing at least one carbon-carbon triple bond, which may be straight or branched, or a combination thereof. Examples of alkynyl include ethynyl, propargyl, 3-methyl-1-pentynyl, and 2- heptynyl, etc. The alkynyl is optionally substituted with one or more halo.

The term "cycloalkyl" refers to a monocyclic or bicyclic saturated carbocyclic ring, in which each ring has 3 to 10 carbon atoms. The "fused analogue" of cycloalkyl refers to a monocyclic ring fused with aryl or heteroaryl, in which the attachment point is on the non-aryl moiety. Examples of cycloalkyl and fused analogues are, for example, cyclopropyl, cyclobutyl, cyclopentyl, cyclohexyl, tetrahydronaphthyl, decahydronaphthyl, and indenyl, etc. The cycloalkyl group is optionally substituted with one or more halogen atoms. The term "heteroalkyl" itself or in combination with another term refers to a stable straight chain or branched chain consisting of at least one carbon atom and at least one heteroatom selected from O, N, P, Si and S or a cyclic hydrocarbon group or a combination thereof, in which the nitrogen atom, phosphorus atom or sulfur atom can be optionally oxidized, and the nitrogen atom can be optionally quaternized. The heteroatom O, N, P, S or Si can be positioned anywhere in the heteroalkyl group or at a position where the alkyl group is connected to the rest of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-, -S(O)-CH₃, -CH₂-CH₂-S(O)₂-CH₃, -CH=CHO-CH₃, -Si(CH₃)₃, -CH₂-CH=N-OCH₃, -CH=CH-N(CH₃)-CH₃, -O-CH₃, -O-CH₂-CH₃ and -CN. At most two or three heteroatoms are contiguous. For example, -CH₂-NH-OCH₃ and -CH₂-O-Si(CH₃)₃. The term "cycloalkoxy" refers to a cycloalkyl group as defined above that is bonded to an oxygen atom, such as cyclopropoxy.

Similarly, the term "heteroalkenyl" itself or in combination with other terms refers to a divalent group derived from a heteroalkyl group, such as, without limitation, -CH₂-CH₂-S-CH₂-CH₂- and -CH₂-S-CH₂-CH₂-CH₂-NH-CH₂-. For heteroalkenyl, the heteroatom can be located at either end or both ends of the chain (for example, alkylene-oxy, alkylene-dioxy, alkylene-amino, alkylene-diamino, etc.). In addition, for groups linked to the alkylene and heteroalkylene, the writing direction of the linking group does not indicate the orientation of the linking group. For example, the molecular formula -C(O) or '- represents -C(O) or '- and - R'OC(O)-. Heteroalkyl, as used herein, includes those groups that are attached to the rest of the molecule through the heteroatom, such as -C(O)R', -C(O)NR', -NR'R', -OR', -SR' and/or - SO₂R'. In the case of referring to "heteroalkyl" and later referring to a specific heteroalkyl such as -NR'R ", it should be understood that the terms heteroalkyl and -NR'R" are not repetitive and mutually exclusive. On the contrary, these specific heteroalkyl groups are cited for clarity. Therefore, the term "heteroalkyl" should not be interpreted herein as excluding specific heteroalkyl groups, such as -NR'R ".

As used herein, the term "substituted heterocyclic ring" or "substituted heterocycloalkyl group" or "substituted heterocyclic group" refers to a heterocyclic group substituted with 1 to 5 (e.g. 1 to 3) substituents. The substituents are the same as those defined for substituted cycloalkyl.

Aromatic ring or aryl refers to an aromatic carbocyclic moiety with one or more closed rings. Examples include, but are not limited to, phenyl, naphthyl, anthracenyl, biphenylyl and pyrenyl.

"Heteroaryl" refers to a monocyclic or bicyclic moiety containing at least one heteroatom selected from oxygen, nitrogen or sulfur, where at least one of the rings is aromatic, and where the one or more rings can be independently fused and/or bridged. Examples include, but are not limited to, pyridyl, pyrrolyl, pyrazolyl, quinolyl, isoquinolyl, indolyl, furyl, thienyl, quinoxalinyl, indazolyl, thieno[2,3-c]pyrazolyl, benzofuryl, thienothiazolyl, benzothiazolyl, oxazolyl, oxadiazolyl, thiadiazolyl, thiazolyl, imidazolyl, triazolyl, tetrazolyl, furyl, triazinyl, thienyl, pyrimidinyl, pyridazinyl, benzoxazolyl, benzothiazolyl, benzimidazolyl, benzofuryl, benzothiophenyl, furyl and [2,3-b]pyridyl, quinolinyl, indolyl, and isoquinolinyl, etc.

Unless otherwise specified, the term "halo" or "halogen" itself or as a moiety of another substituent refers to a fluorine, chlorine, bromine or iodine atom. In addition, the term "haloalkyl" is intended to include monohaloalkyl and polyhaloalkyl. For example, the term "halo(C₁-C₄)alky" is intended to include, without limitation, trifluoromethyl, 2,2,2-trifluoroethyl, 4- chlorobutyl, and 3- bromopropyl, etc.

Optical isomers, diastereomers, geometric isomers and tautomers: Some compounds of Formula I may contain one or more ring systems, so cis and trans isomers may exist. The present invention is intended to cover all these cis and trans isomers. A compound containing an olefin double bond means that the compound includes an E and Z geometric isomer, unless otherwise specified.

Some compounds described herein can have one or more asymmetric centers, and can be used as racemates and racemic mixtures, single enantiomers, diastereomeric mixtures and single diastereomers.

Some compounds described herein may have different point of attachment of hydrogen atoms, which are called tautomers. Examples with this regard may be ketone and enol forms called keto-enol tautomers. Individual tautomers and their mixtures are embraced by the compound of Formula I.

The compound of Formula I can be separated into diastereomers and enantiomers, for example, by separation and crystallization in a suitable solvent (such as methanol or ethyl acetate or a mixture thereof). A pair of enantiomers thus obtained can be separated into single stereoisomers by a conventional method, for example, using an optically active amine or acid as a resolving reagent or in a chiral **HPLC** column.

Any enantiomer of the compound of Formula I can be obtained by stereospecific synthesis using an optically pure starting material or a reagent with a known configuration.

In addition, the compound of Formula I can also include a series of stable isotope-labeled analogues. For example, one or more protons in the compound of Formula I can be replaced by deuterium atom(s), thus providing a deuterated analogue with improved pharmacological activities.

"Pharmaceutically acceptable salt" refers to an acidic salt or basic salt of the compound of the present invention, which has the required pharmacological activity and is not harmful biologically or otherwise. The salt may include, but is not limited to, acetate, adipate, benzoate, citrate, camphorate, camphorsulfonate, digluconate, dodecylsulfate, ethanesulfonate, fumarate, glucoheptonate, glycerophosphate, hemisulfate, heptanoate, caproate, hydrobromide, hydrochloride, hydroiodide, 2- hydroxyethansulfonate, lactate, maleate, and oxalate. It should be understood that within the scope of the present invention, the above technical features of the present invention and the technical features described in detail below (for example in the examples) can be combined with each other to form a new or preferred technical solution.

In an embodiment, the present invention discloses a compound of Formula I, where R, G, W¹, W², W³, X and Y are as defined herein, and a pharmaceutically acceptable salt, solvate and composition thereof.

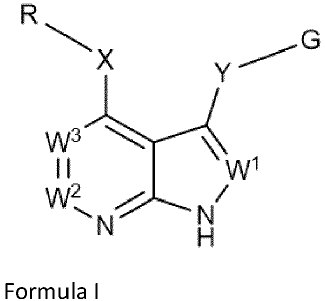

In Formula I,
R is independently selected from a substituted or unsubstituted alkyl, an alkenyl group, an alcohol in which the alkyl group is unsubstituted or substituted, an amine in which the alkyl group is unsubstituted or substituted, a substituted or unsubstituted four to seven-membered carbocyclic ring, and a substituted or unsubstituted four to seven-membered carbocyclic ring having a heteroatom including, but not limited to, N, S, and O.

X is independently selected from O, NR, NH, and CO.

W¹, W², and W³ are independently selected from CH or N that satisfies the valence bond theory.

Y is independently selected from carbonyl, methylene, difluoromethyl, and a structure shown below, without limitation:
O, NH,

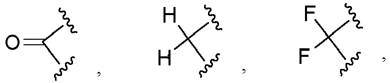

When Y is absent, a bond exists between the unit G and the heteroaryl ring of the selected parent nucleus of Formula I.

R-X is independently selected from, but is not limited to, the following structures:

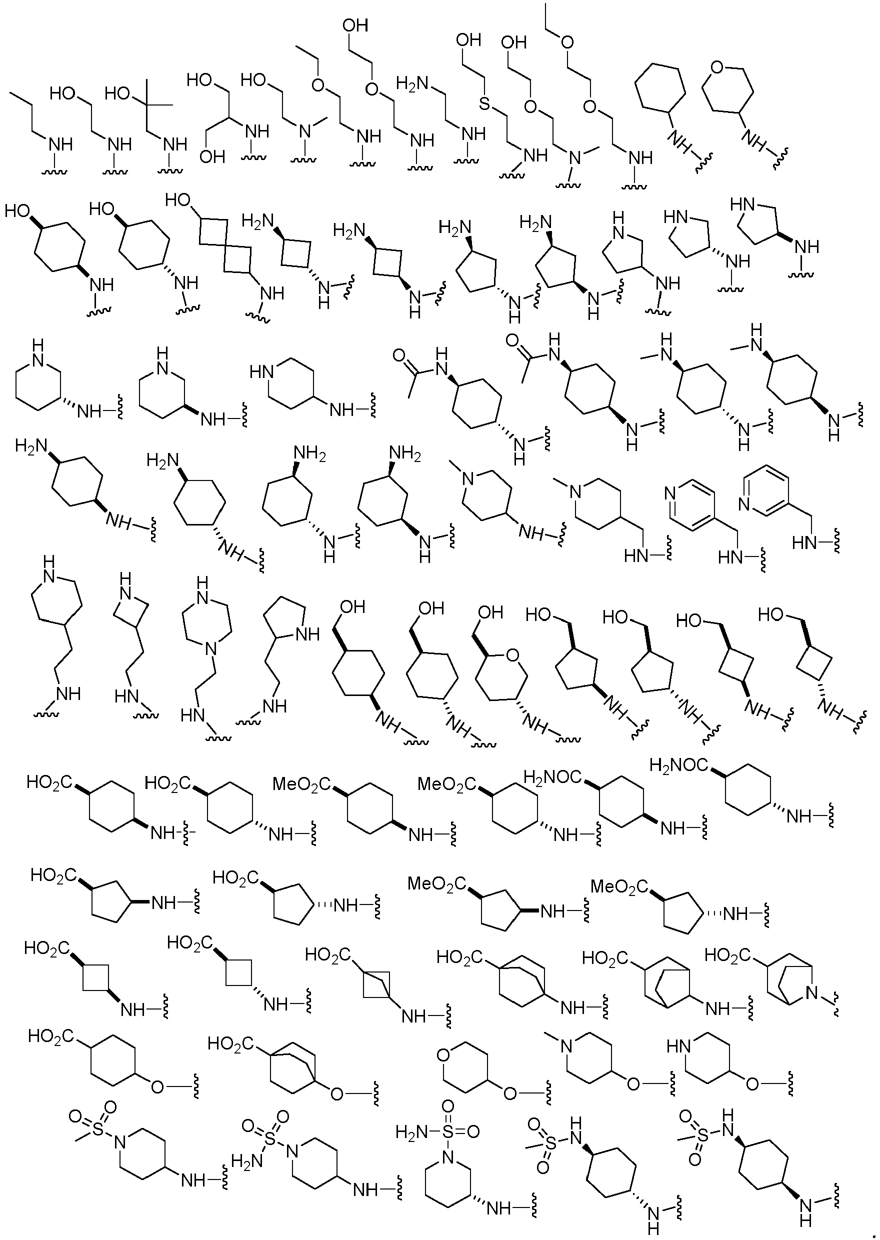

G is independently selected from a 5 or 6-membered aryl, a substituted aryl, a heteroaryl, a substituted heteroaryl containing 1 to 3 heteroatoms selected from N, O and S, and biphenylyl, where the aryl and heteroaryl are optionally substituted with R¹, R² or R³. Further, G is independently selected from, but is not limited to, the following structures:

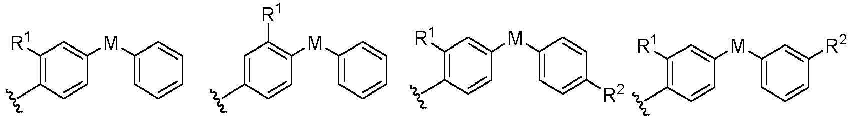

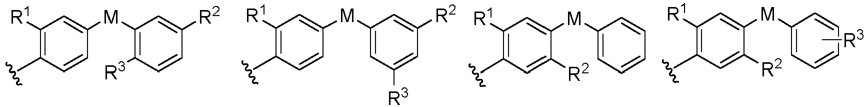

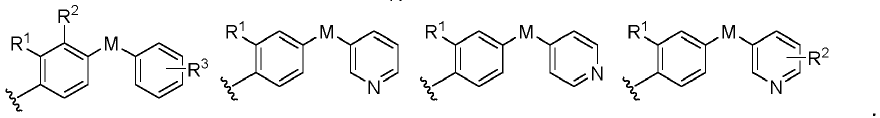

In these structures, R¹, R², and R³ are independently selected from hydrogen, amine, hydroxyl, halo, C₁-C₆ alkyl, C₁-C₆ alkoxy optionally substituted with one or more alkoxy, C₁-C₆ haloalkyl, C₁-C₆ haloalkoxy, amine, alkylamine, amide, ether, thioether, a heterocarbocyclic compound containing one or two 4- to 6-membered ring and a carbocyclic ring and having a heteroatom that is N or O, phenoxy, substituted phenoxy. The intermediate skeleton atoms of the ring together form a C₃-C₈ carbocyclic or 4-8-membered heterocyclic ring, in which the heteroatom in the heterocyclic ring is selected from: S, O or NR; R is H, C₁-C₁₀ alkyl, C₃-C₁₀ cycloalkyl, or C₆-C₂₀ aryl.

M is independently selected from -O-, -NH-, -CH₂-, -CO-, -CONH-, -S-, and -SO₂-.

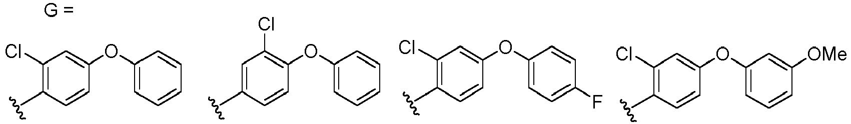

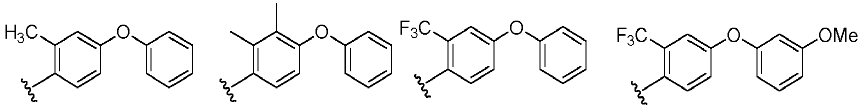

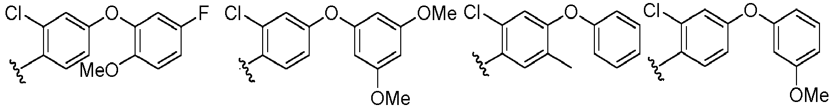

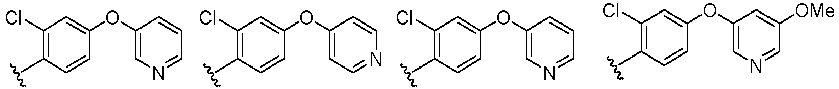

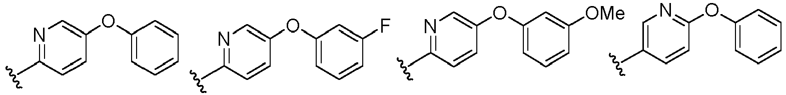

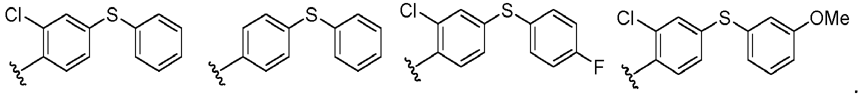

In some embodiments, the compound has Formulas **Ia, Ib,** and **Ic,** and an enoate salt or a pharmaceutically acceptable salt and a solvate thereof.

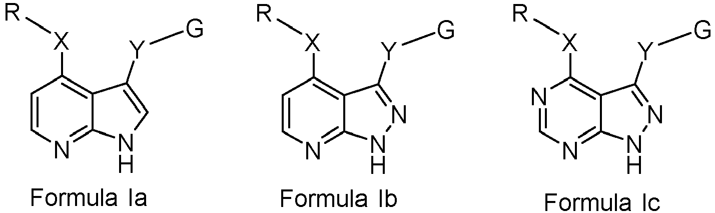

R is independently selected from a substituted or unsubstituted C₁₋₁₀ alkyl, alkenyl, alkyl or a substituted alcohol, alkyl or a substituted amine, a substituted or unsubstituted four- to seven-membered carbocyclic ring, a substituted or unsubstituted four- to seven-membered carbocyclic ring having a heteroatom including N, S, or O.

X is independently selected from O, NR, NH, and CO.

Y is independently selected from, but is not limited to, carbonyl, methylene, difluromethyl and the following structures:

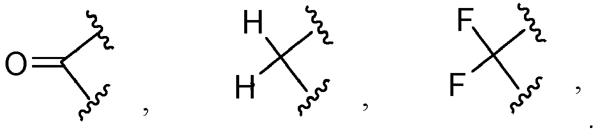

When Y is absent, a chemical bond exists between the unit G and the heteroaryl ring of the selected parent nucleus of Formula I.

R-X is independently selected from a list below, as described for Formula I.

G is independently selected from a substituted or unsubstituted 5- or 6-membered aryl, a substituted heteroaryl containing 1 to 3 heteroatoms selected from N, O and S, and biphenylyl, in which the aryl and heteroaryl are optionally substituted with R¹, R² or R³. Further, G is independently selected from the following structure, as described for Formula I, but is not limited to:

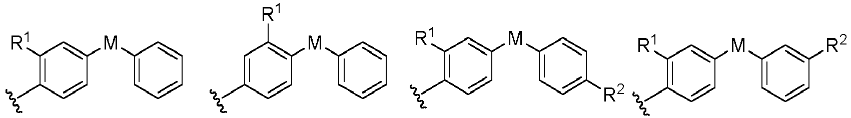

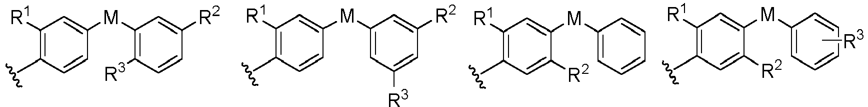

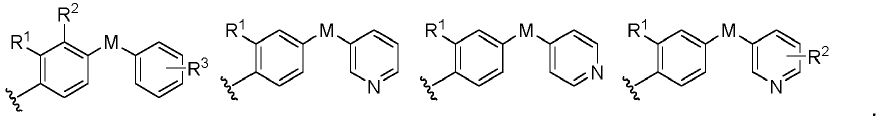

In these structures, R¹, R² or R³ is independently selected from hydrogen, amine, hydroxyl, halo, C₁-C₆ alkyl, C₁-C₆ alkoxy optionally substituted with one or more alkoxy, C₁-C₆ haloalkyl, C₁-C₆ haloalkoxy, amine, alkylamine, amide, ether, thioether, a heterocyclic ring having one or two 1 to 6 membered rings or carbocyclic rings, a heterocarbocyclic ring having a heteroatom that is N or O, phenoxy, and substituted phenoxy. The intermediate skeleton atoms of the ring together form a C₃-C₆ carbocyclic or 4-8-membered heterocyclic ring, in which the heteroatom in the heterocyclic ring is selected from: S, O or NR; R is H, C₁-C₁₀ alkyl, C₃-C₁₀ cycloalkyl, or C₆-C₂₀ aryl.

M is independently selected from -O-, -NH-, -CH₂-, -CO-, -CONH-, -S-, and -SO₂-.

In some embodiments, the compound has Formulas Id, le, and If, and an enoate salt or a pharmaceutically acceptable salt and a solvate thereof.

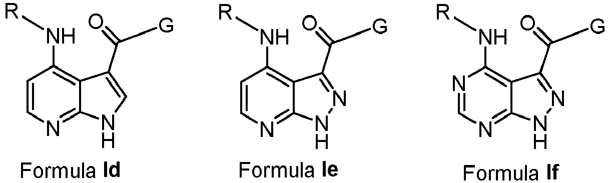

R is independently selected from substituted or unsubstituted C₁₋₁₀ alkyl, an alkenyl group, an alcohol in which the alkyl group is unsubstituted or substituted, an thiol in which the alkyl group is unsubstituted or substituted, a substituted or unsubstituted four to seven-membered carbocyclic ring, and a substituted or unsubstituted four to seven-membered carbocyclic ring having a heteroatom including, but not limited to, N, S, and O.

R is independently selected from, but is not limited to, the following structures:

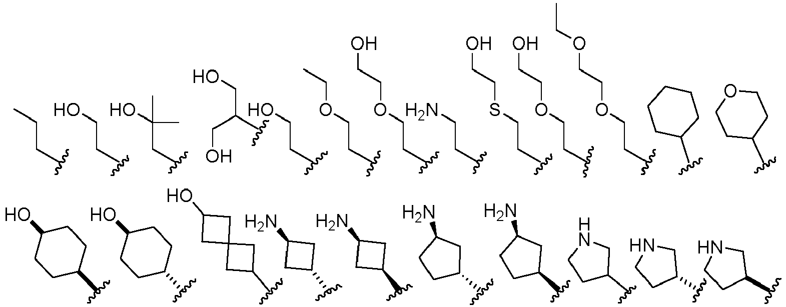

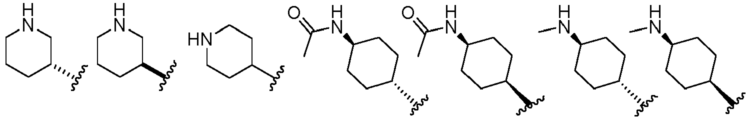

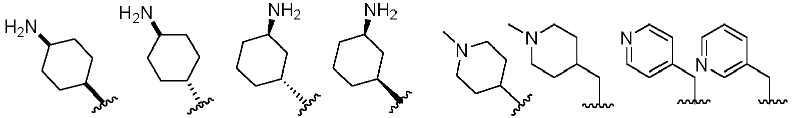

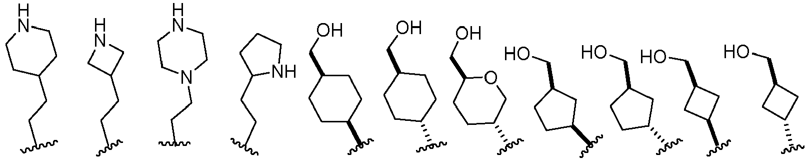

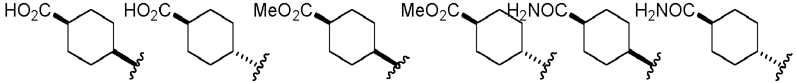

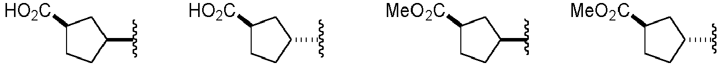

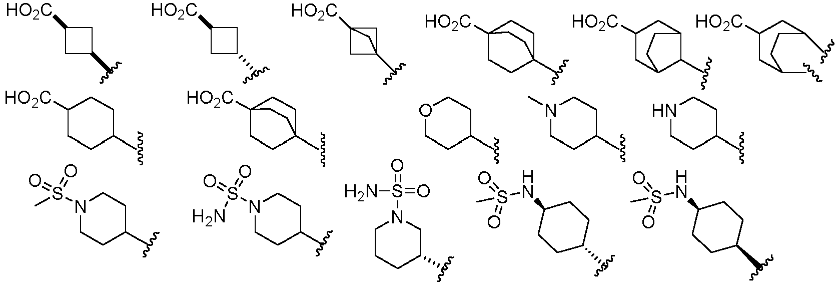

G is independently selected from a substituted or unsubstituted 5- or 6-membered aryl, a substituted aryl, a substituted heteroaryl containing 1 to 3 heteroatoms selected from N, O and S, and biphenylyl, in which the aryl and heteroaryl are optionally substituted with R¹, R² or R³. R¹, R² or R³ is independently selected from hydrogen, amine, hydroxyl, halo, C₁-C₆ alkyl, C₁-C₆ alkoxy optionally substituted with one or more alkoxy, C₁-C₆ haloalkyl, C₁-C₆ haloalkoxy, amine, alkylamine, amide, ether, thioether, a heterocyclic ring having one or two 1 to 6 membered rings or carbocyclic rings, a heterocarbocyclic ring having a heteroatom that is N or O, phenoxy, and substituted phenoxy. The intermediate skeleton atoms of the ring together form a C₃-C₆ carbocyclic or 4-8-membered heterocyclic ring, in which the heteroatom in the heterocyclic ring is selected from: S, O or NR; R is H, C₁-C₁₀ alkyl, C₃-C₁₀ cycloalkyl, or C₆-C₂₀ aryl.

G is independently selected from, but is not limited to, the list as described for Formula I.

### Examples 1-90:

### General description

Silica gel (100-200) and various eluants were used for column chromatography. The solvent was removed using **Buchii** rotary evaporator or **Genevac** centrifugal evaporator. Preparative **LC/MS** was conducted with an acidic mobile phase using **Waters** automatic purifier and 19 x 100mm XTerra 5 micron **MS CI8** column. The nuclear magnetic resonance spectrum was recorded by Varian 400MHz spectrometer. When the term "inert" is used to describe a reactor (e.g., reaction vessel, flask, and glass reactor, etc.), it means that the air in the reactor has been replaced by an inert gas (e.g., nitrogen, and argon, etc.) that is substantially free of water or dry.

The following abbreviations are used here:
Definition: The following abbreviations have the following meanings:
HATU: 2-(7-azabenzotriazol-1-yl)-N, N, N', N'-tetramethyl uronium hexafluorophosphate DPCI: N, N'-diisopropyl carbodiimide
DIEA: N, N-diisopropylethyl amine
TEA: triethylamine
DMAP: 4-dimethylaminopyridine
DMF: N,N-dimethyl formamide
NMP: N-methyl pyrrolidine
THF: tetrahydrofuran
DCM: dichloromethane
TFA: trifluoroacetic acid
DMA: N, N-dimethylacetaimide
TLC: thin layer chromatography
TMOF: trimethyl orthoformate
PTSA: p-toluenesulfonic acid
NIS: N-iodosuccinimide
eq: equivalent
mmol: millimole
mol: mole
mL: milliliter
L: liter
MHz: megahertz
δ: chemical shift
DMSO-d6: deuterated dimethyl sulfoxide
Hrs, hr, h: hour
Ms: mass spectrometry
M/z: mass-to-charge ratio

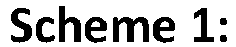

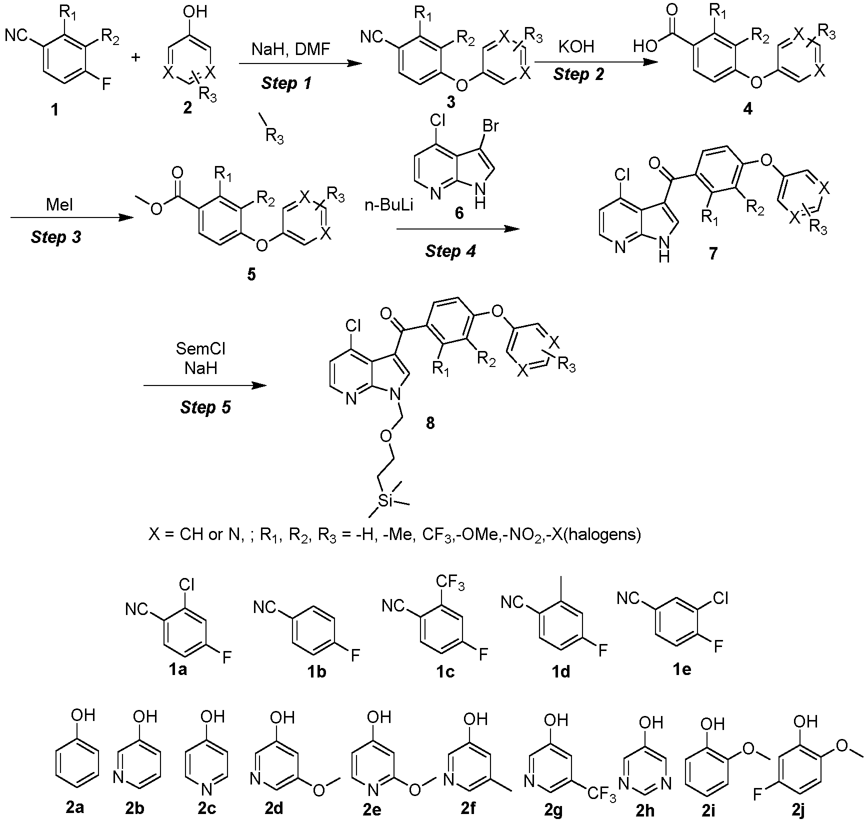

Compounds (**8a-8n**, Table 1) were prepared according to Scheme **1** and following the procedure for preparing **8a**.

### Preparation of (4-chloro-1-((2-(trimethylsilyl)ethoxy)methyl)-1H-pyrrolo[2,3-b]pyridin-3-yl)(2-chloro-4-phenoxyphenyl)methanone (8a)

Step 1: Under nitrogen atmosphere, a mixed solution of Compound **2a** (25.5 g, 0.27 mol) in DMF was cooled to about 0°C. NaH (18.0 g, 0.452 mol, 60% in oil) was added batchwise to the mixed solution and stirred for 30 min. Compound **1a** (35 g, 0.226 mol) was added batchwise to the mixed solution. The reaction mixture was heated to room temperature and stirred for 4 hrs, until TLC showed that Compound **1a** was completely consumed. The reaction mixture was quenched with a saturated ammonium chloride solution at 0°C, and extracted with ethyl acetate (200 ml X 3). The organic phase was washed with water and brine, combined, dried over anhydrous sodium sulfate, and evaporated to obtain a dry product (52 g, 100%). Product **3a** could be used without further purification since the purity met the use in the next step. ¹H NMR(DMSO-d6, 400 MHz):δ=7.95(d,1H),7.52-7.48(m,2H),7.34-7.30(m,2H),7.21-7.19(m,2H),7.05-7.02(m,1H).

Step 2: KOH (228 g, 4.09 mol) was added to a mixed solution of Compound **3a** (52 g, 0.227 mol) in EtOH (200 ml) and H₂O (180 ml). The reaction mixture was stirred at 90°C overnight, until TLC showed that Compound **3a** was completely consumed. Then, the reaction mixture was cooled to room temperature, and distilled to remove most of ethanol. The system was adjusted to pH 4-5 with 2 N hydrochloric acid, and a solid was precipitated out. The solid was filtered, collected, and subjected to 5 rounds of rotary evaporation with toluene to remove water. A crude product **4a** (57g, 100%) was obtained, which was directly used in the next step without purification. ¹H NMR (DMSO-d6, 400 MHz): δ =7.88 (d, 1H), 7.50-7.46 (m, 2H), 7.29-7.27 (m, 1H), 7.17-7.15 (m, 2H), 7.08 (d, 1H), 6.98-6.95 (m, 1H).

Step 3: Under nitrogen atmosphere, a solution of Compound **4a** (57 g, 0.257 mol) and K₂CO₃ (106 g, 0.771 mol) in DMF (360 ml) was cooled to 0°C. lodomethane (40g, 0.283mol) was added dropwise to the reaction mixture. Then the reaction mixture was heated to room temperature and further reacted for 4-6 hrs with stirring until TLC showed that Compound **4a** was completely consumed. The reaction mixture was added with water and extracted with ethyl acetate (200 ml x 3). The organic phase was washed with water and brine, and the organic layers were combined, dried over Na₂SO₄ and evaporated to obtain a crude product. The crude product was purified by flash chromatography using a mixed solvent of PE and EAs an eluant, to obtain an oil **5a**(40 g, yield 67%). ¹H NMR (DMSO-d6, 400 MHz): δ =7.88 (d, 1H), 7.50-7.46 (m, 2H), 7.30-7.28 (m, 1H), 7.17-7.15 (m, 2H), 7.11 (d, 1H), 6.99-6.97 (m, 1H), 3.84 (s, 3H).

Step 4: Under argon atmosphere, a solution of Compound **6** (4.0 g, 17.3 mmol) in THF (50 mL) was cooled to -78°C. n- butyl lithium (1.6M in hexane, 23 mL, 36.3 mmol) was added dropwise to the mixed solution. The mixed solution was continuously reacted at -78°C with stirring for 1 hr, and then a solution of Compound **5a** (4.8 g, 18.1 mmol) in THF (15 ml) precooled to -78°C was added. The reaction mixture was stirred at -78°C for 3-5 hrs, until TLC showed that Compound **6** was completely consumed, and then the reaction was quenched with 1 N HCl. The reaction mixture was heated to room temperature, extracted with EA, and washed with saturated brine. The organic phase was dried over anhydrous Na₂SO₄ and evaporated to obtain a crude product. The crude product was recrystallizedin ethyl acetate, to obtain Compound **7a** (2.9 g, yield: 43%). ¹H NMR (DMSO-d6, 400 MHz): δ = 12.96 (s, 1H), 8.31 (d, 1H), 7.97 (s, 1H), 7.56 (d, 1H), 7.50-7.46 (m, 2H), 7.38-7.37 (m, 1H), 7.27-7.24 (m, 1H), 7.19-7.15 (m, 3H), 7.02-7.00 (m, 1H).

Step 5: Under nitrogen atmosphere, a solution of Compound **7a** (2.8 g, 7.3 mmol) in THF (35 mL) was cooled to 0°C. NaH (0.35 g, 8.7 mmol, 60% in oil) was added batchwise to the solution. Then, the mixture was continuously stirred for 30min, and then SEMCI (1.8 g, 10.9 mmol) was added dropwise. The reaction mixture was heated to room temperature and further stirred for 3-5 hrs, until TLC showed that Compound **7a** was completely consumed. The reaction was quenched with saturated NH₄Cl at 0°C, and extracted with ethyl acetate (20 ml x 3). The organic layers were washed with water and saturated brine and combined. The organic phase was dried over anhydrous Na₂SO₄ and evaporated to obtain a crude product, which was purified by column chromatography on silica gel to obtain Compound **8a** (2.1 g, yield: 57%). ¹H NMR (DMSO-d6, 400 MHz): δ = 8.38 (d, 1H), 8.20 (s, 1H), 7.49 (d, 1H), 7.46-7.44 (m, 3H), 7.28-7.24 (m, 1H), 7.18-7.15 (m, 3H), 7.02-7.00 (m, 1H), 5.67 (s, 2H), 3.55-3.51 (m, 2H), 0.81-0.77 (m, 2H), 0.12 (s, 9H).

**Table 1. Intermediate and analysis data**

| | |
|---|---|
| 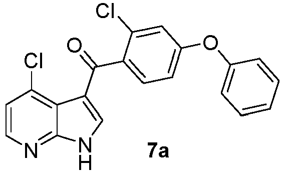 (4-chloro-1H-pyrrolo[2,3-b]pyridin-3-yl)(2-chloro-4-phenoxyphenyl)methanone | 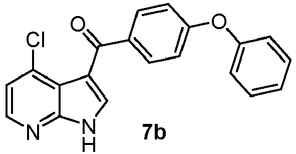 (4-chloro-1H-pyrrolo[2,3-b]pyridin-3-yl)(4-phenoxyphenyl)methanone |
| MS m/z: 383.0 (M+H)⁺,¹H NMR (DMSO-d6, 400 MHz): δ = 8.31 (d, 1H), 7.97 (s, 1H), 7.56 (d, 1H), 7.50-7.46 (m, 2H), 7.37 (d, 1H), 7.27-7.24(m, 1H), 7.19-7.15 (m, 3H), 7.02-7.00 (m, 1H). | MS m/z: 349.1 (M+H)⁺,¹H NMR (DMSO-d6, 400 MHz): δ = 8.30 (d, 1H), 8.04 (s, 1H), 7.88 (d, 1H), 7.49-7.45 (m, 2H), 7.33 (d, 1H), 7.26-7.23 (m, 1H), 7.16 (d, 2H), 7.08 (d, 2H). |
| 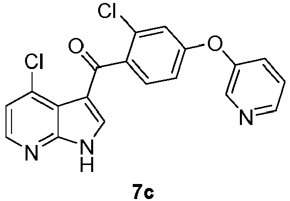 (4-chloro-1H-pyrrolo[2,3-b]pyridin-3-yl)(2-chloro-4-(pyridin-3-oxy)phenyl)methanone | 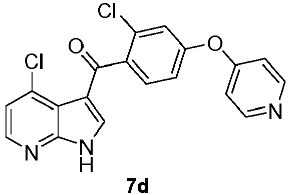 (4-chloro-1H-pyrrolo[2,3-b]pyridin-3-yl)(2-chloro-4-(pyridin-4-oxy)phenyl)methanone |
| MS m/z:384.0 (M+H)⁺, | MS m/z:384.0 (M+H)⁺, |
| 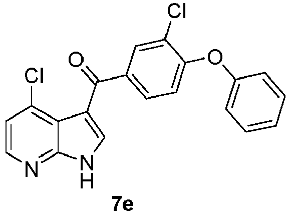 (4-chloro-1H-pyrrolo[2,3-b]pyridin-3-yl)(3-chloro-4-phenoxyphenyl)methanone | 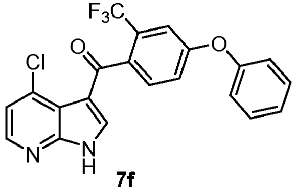 (4-chloro-1H-pyrrolo[2,3-b]pyridin-3-yl)(4-phenoxy-2-(trifluoromethyl)phenyl)methanone MS m/z:417.1 (M+H)⁺,¹H NMR (DMSO-d6, 400 MHz): δ = 8.31 (d, 1H), 7.98 (s, 1H), 7.64 (d, 1H), 7.51-7.47 (m, 2H), 7.43-7.42 (m, 1H), 7.38 (d, 1H), 7.29-7.27 (m, 2H), 7.25-7.20 (m, 2H). |
| MS m/z:383.0 (M+H)⁺, | |
| 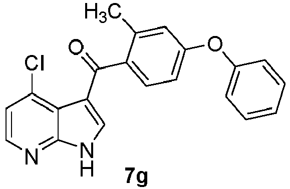 (4-chloro-1H-pyrrolo[2,3-b]pyridin-3-yl)(2-methyl-4-phenoxyphenyl)metha none | 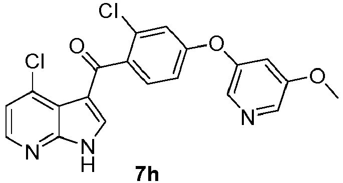 (4-chloro-1H-pyrrolo[2,3-b]pyridin-3-yl)(2-chloro-4-((5-methoxypyridin-3-yl)oxy)phenyl)methanone |
| MS m/z:363.1 (M+H)⁺, | |
| 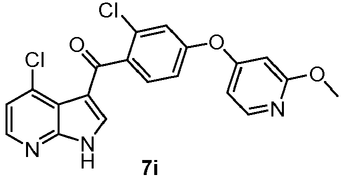 | 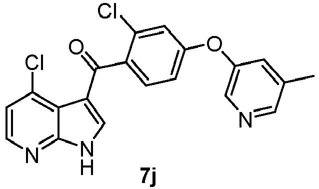 |
| (4-chloro-1H-pyrrolo[2,3-b]pyridin-3-yl)(2-chloro-4-((2-methoxypyridin-4-(oxy)phenyl)methanone | (4-chloro-1H-pyrrolo[2,3-b]pyridin-3-yl)(2-chloro-4-((5-methylpyridin-3-yl)oxy)phenyl)methanone |
| MS m/z:414.0 (M+H)⁺, | MS m/z: 398.0 (M+H)⁺, |
| 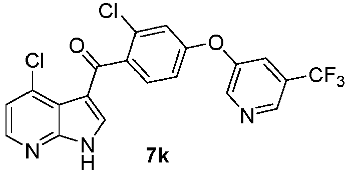 (4-chloro-1H-pyrrolo[2,3-b]pyridin-3-yl)(2-chloro-4-((5-(trifluoromethyl)pyridin-3)-(oxy)phenyl)methanone | 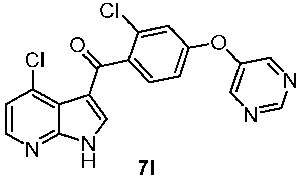 (4-chloro-1H-pyrrolo[2,3-b]pyridin-3-yl)(2-chloro-4-(pyrimidin-5-oxy)phenyl)methanone |
| MS m/z:452.0 (M+H)⁺, 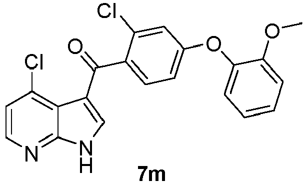 (4-chloro-1H-pyrrolo[2,3-b]pyridin-3-yl)(2-chloro-4-(2-methoxyphenoxy)phenyl)methanone | MS m/z: 385.0 (M+H)⁺, 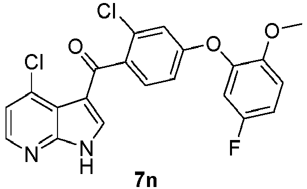 (4-chloro-1H-pyrrolo[2,3-b]pyridin-3-yl)(2-chloro-4-(5-fl uoro-2-methoxyphenoxy)phenyl)methanone |
| MS m/z: 413.0 (M+H)⁺, | MS m/z: 431.0 (M+H)⁺, |
| 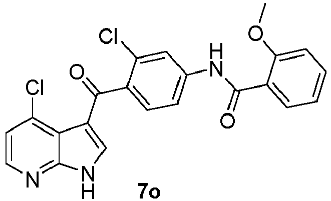 N-(3-chloro-4-(4-chloro-1H-pyrrolo[2,3-b]pyridin-3-carbonyl)phenyl)-2-methoxybenzamide | 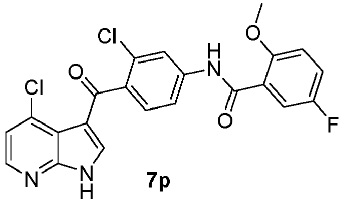 N-(3-chloro-4-(4-chloro-1H-pyrrolo[2,3-b]pyridin-3-carbonyl)phenyl)-5-fluoro-2-methoxybenzamide |
| MS m/z: 440.1 (M+H)⁺, | MS m/z: 458.0 (M+H)⁺, |
| 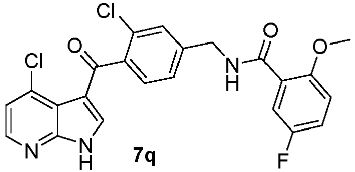 N-(3-chloro-4-(4-chloro-1H-pyrrolo[2,3-b]pyridin-3-carbonyl)benzyl)-5-fluoro-2-methoxybenzamide MS m/z: 472.1 (M+H)⁺, | 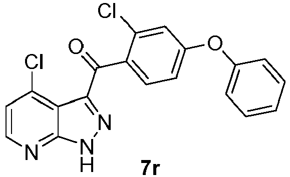 (4-chloro-1H-pyrazolo[3,4-b]pyridin-3-yl)(2-chloro-4-phenoxyphenyl)methanone MS m/z: 384.0 (M+H)⁺, |
| 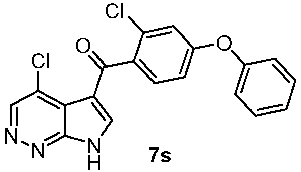 (2-chloro-4-phenoxyphenyl)(4-chloro-7H-pyrrolo[2,3-c]pyridazin-5-yl)methanone MS m/z: 384.0 (M+H)⁺, | 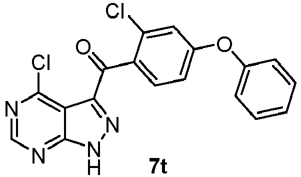 (4-chloro-1H-pyrazolo[3,4-d]pyrimidin-3-yl)(2-chloro-4-phenoxyphenyl)methanone MS m/z: 385.0 (M+H)⁺, |
| 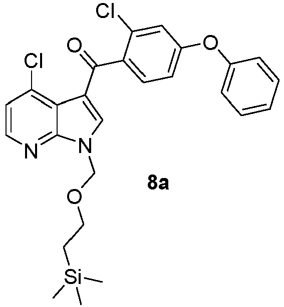 (4-chloro-1-((2-(trimethylsilyl)ethoxy)methyl)-1H-pyrrolo[2,3-b]pyridi n-3-yl)(2-chloro-4-phenoxyphenyl)methanone MS m/z:513.1 (M+H)⁺, ¹H NMR (DMSO-d6, 400 MHz): δ = 8.38 (d, 1H), 8.20 (s, 1H), 7.49 (d, 1H), 7.46-7.44 (m, 3H), 7.28-7.24 (m, 1H), 7.18-7.15 (m, 3H), 7.02-7.00 (m, 1H), 5.67 (s, 2H), 3.55-3.51 (m, 2H), 0.81-0.77 (m, 2H), 0.12 (s, 9H). | 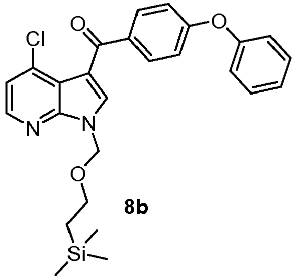 (4-chloro-1-((2-(trimethylsilyl)ethoxy)methyl)-1H-pyrrolo[2,3-b]pyridin-3-yl)(4-phenoxyphenyl)methanone MS m/z:479.2 (M+H)⁺, ¹H NMR (DMSO-d6, 400 MHz): δ = 8.40 (d, 1H), 8.39 (s, 1H), 7.98 (d, 1H), 7.60-7.56 (m, 2H), 7.51 (d, 1H), 7.37-7.34 (m, 1H), 7.25 (d, 2H), 7.18 (d, 2H), 5.81 (s, 2H), 3.70-3.66 (m, 2H), 0.95-0.91 (m, 2H), 0.01 (s, 9H). |
| 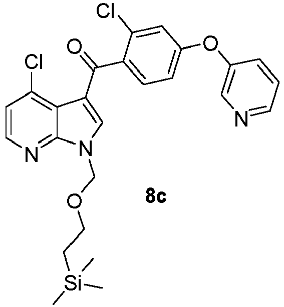 (4-chloro-1-((2-(trimethylsilyl)ethoxy)methyl)-1H-pyrrolo[2,3-b]pyridin-3-yl)(2-chloro-4)-(pyridin-3-oxy)phenyl)methanone MS m/z: 514.1 (M+H)⁺ | 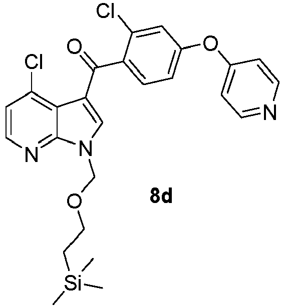 (4-chloro-1-((2-(trimethylsilyl)ethoxy)methyl)-1H-pyrrolo[2,3-b]pyridin-3-yl)(2-chloro-4-(pyridin-4-oxy)phenyl)methanone MS m/z:514.1 (M+H)⁺ |
| 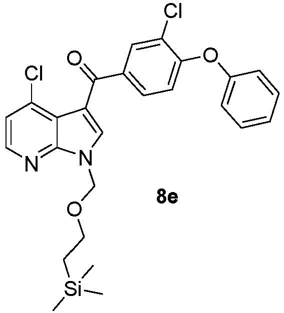 (4-chloro-1-((2-(trimethylsilyl)ethoxy)methyl)-1H-pyrrolo[2,3-b]pyridin-3-yl)(3-chloro-4-phenoxyphenyl)methanone MS m/z:513.1 (M+H)⁺ | 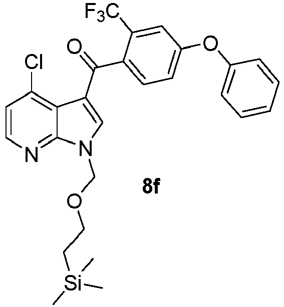 (4-chloro-1-((2-(trimethylsilyl)ethoxy)methyl)-1H-pyrrolo[2,3-b]pyridin-3-yl)(4-phenoxy-2)-(trifluoromethyl)phenyl)methanone MS m/z:547.1 (M+H)⁺;¹H NMR (DMSO-d6, 400 MHz): δ = 8.50 (d, 1H), 8.35 (s, 1H), 7.77 (d, 1H), 7.64-7.62 (m, 2H), 7.60-7.57 (m, 2H), 7.42-7.39 (m, 2H), 7.32 (d, 2H), 5.80 (s, 2H), 3.67-3.63 (m, 2H), 0.94-0.90 (m, 2H), 0.01 (s, 9H). |
| 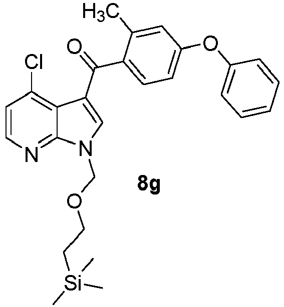 (4-chloro-1-((2-(trimethylsilyl)ethoxy)methyl)-1H-pyrrolo[2,3-b]pyridi n-3-yl)(2-methyl-4-phenoxyphenyl)methanone. MS m/z:593.2 (M+H)⁺ | 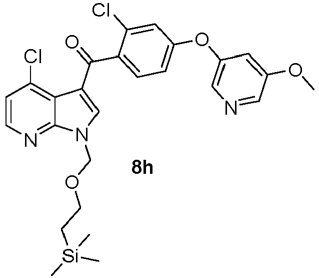 (4-chloro-1-((2-(trimethylsilyl)ethoxy)methyl)-1H-pyrrolo[2,3-b]pyridin-3-yl)(2-ch loro-4-((5-methoxypyridin-3-yl)oxy)phenyl)methanone. MS m/z:544.1 (M+H)⁺ |
| 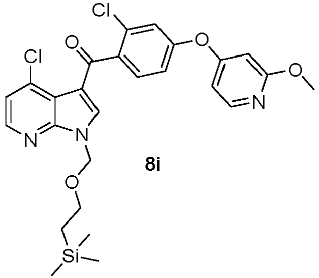 (4-chloro-1-((2-(trimethylsilyl)ethoxy)methyl)-1H-pyrrolo[2,3-b]pyridi n-3-yl)(2-ch loro-4-((2-methoxypyridin-4-yl)oxy)phenyl)methanone. MS m/z:544.1 (M+H)⁺ | 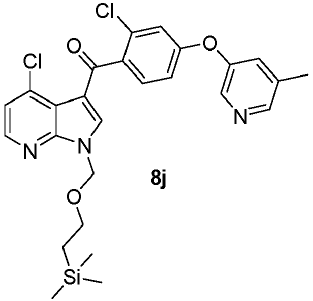 (4-chloro-1-((2-(trimethylsilyl)ethoxy)methyl)-1H-pyrrolo[2,3-b]pyridin-3-yl)(2-chloro-4-((5-methylpyridin-3-yl)oxy)phenyl)methanone. MS m/z:528.1 (M+H)⁺ |
| 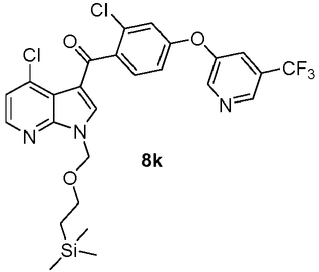 (4-chloro-1-((2-(trimethylsilyl)ethoxy)methyl)-1H-pyrrolo[2,3-b]pyridi n-3-yl)(2-chloro-4-((5-(trifluoromethyl)pyridin-3-yl)-oxy)phenyl)methanone. MS m/z:582.1 (M+H)⁺ | 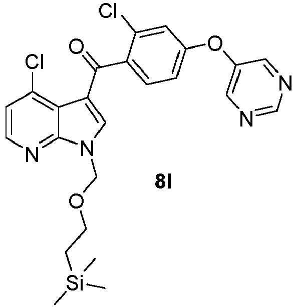 (4-chloro-1-((2-(trimethylsilyl)ethoxy)methyl)-1H-pyrrolo[2,3-b]pyridin-3-yl)(2-chloro-4-(pyrimidin-5-ethoxy)phenyl)methanone. MS m/z:515.1 (M+H)⁺ |
| 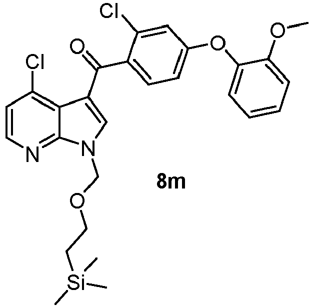 (4-chloro-1-((2-(trimethylsilyl)ethoxy)methyl)-1H-pyrrolo[2,3-b]pyridi n-3-yl)(2-ch loro-4-(2-methoxyphenoxy)phenyl)methanone. MS m/z:543.1 (M+H)⁺ | 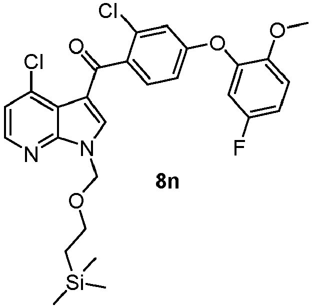 (4-chloro-1-((2-(trimethylsilyl)ethoxy)methyl)-1H-pyrrolo[2,3-b]pyridin-3-yl)(2-chloro-4-(5-fluoro-2-methoxyphenoxy)phenyl)methanone. MS m/z:561.1 (M+H)⁺ |
| 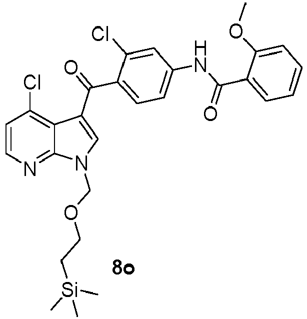 N-(3-chloro-4-(4-chloro-1-((2-(trimethylsilyl)ethoxy)methyl)-1H-pyrrolo[2,3-b]pyridi n-3-carbonyl)phenyl)-2-methoxybenzamide. MS m/z:570.1 (M+H)⁺ | 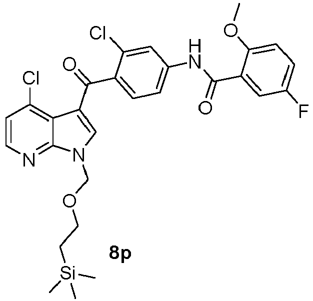 N-(3-chloro-4-(4-chloro-1-((2-(trimethylsilyl)ethoxy)methyl)-1H-pyrrolo[2,3-b]pyridin-3-carbonyl)phenyl)-5-fluoro-2-methoxybenzamide. MS m/z:588.1 (M+H)⁺ |
| 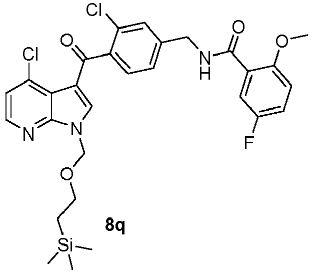 N-(3-chloro-4-(4-chloro-1-((2-(trimethylsilyl)ethoxy)methyl)-1H-pyrrolo[2,3-b]pyridin-3-carbonyl)benzyl)-5-fluoro-2-methoxybenzamide . MS m/z:602.1 (M+H)⁺ | 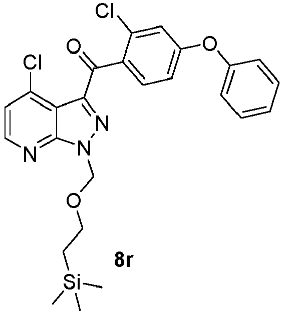 (4-chloro-1-((2-(trimethylsilyl)ethoxy)methyl)-1H-pyrazolo[3,4-b]pyridin-3-yl)(2-chloro-4-phenoxyphenyl)methanone. MS m/z:514.1 (M+H)⁺ |
| 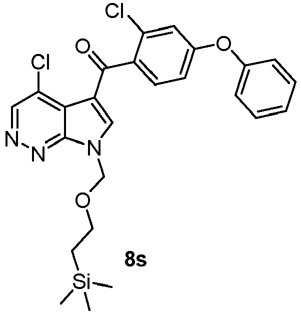 (2-chloro-4-phenoxyphenyl)(4-chloro-7-((2-(trimethylsilyl)ethoxy)methyl)-7H-pyrrolo[2,3-c]pyridazi n-5-yl)metha none. MS m/z:514.1 (M+H)⁺ | 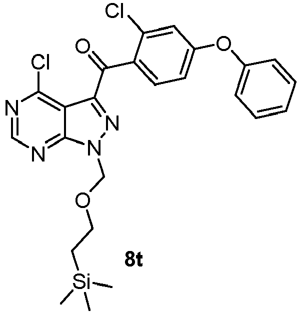 (4-chloro-1-((2-(trimethylsilyl)ethoxy)methyl)-1H-pyrazolo[3,4-d]pyrimidin-3-yl)(2-chloro-4-phenoxyphenyl)methanone. MS m/z:515.1 (M+H)⁺ |

Similarly, Intermediates **8o-8q** (Table 1) were prepared according to Schemes **2** and **3** following known methods in the literatures: **8o-8q** (WO2004056830), **8r-8t** (MonatshChem 148305 314 2017).

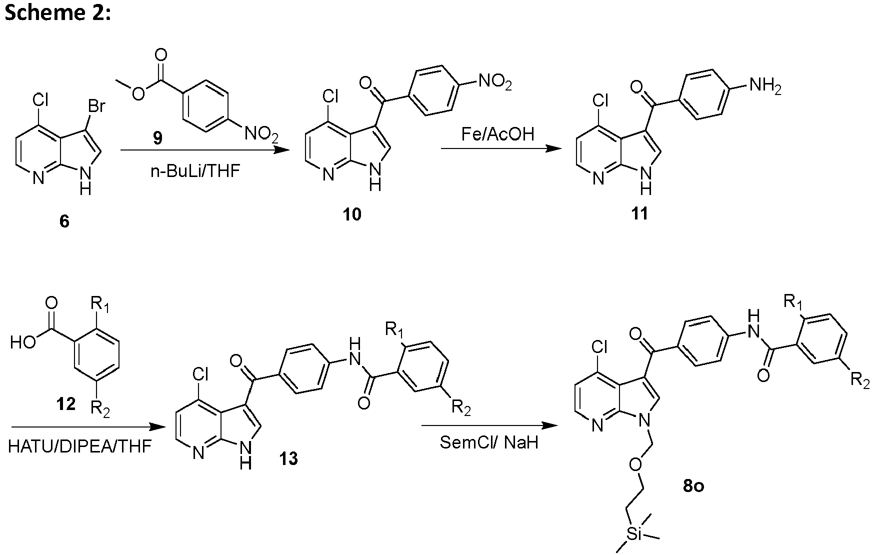

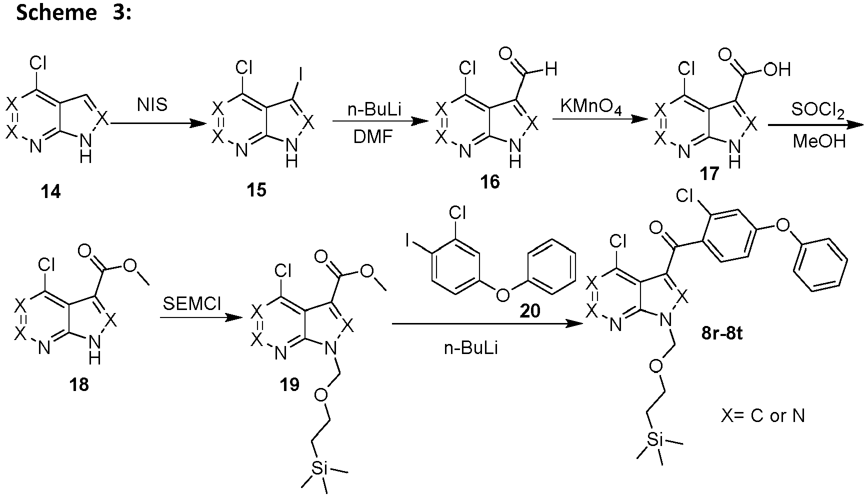

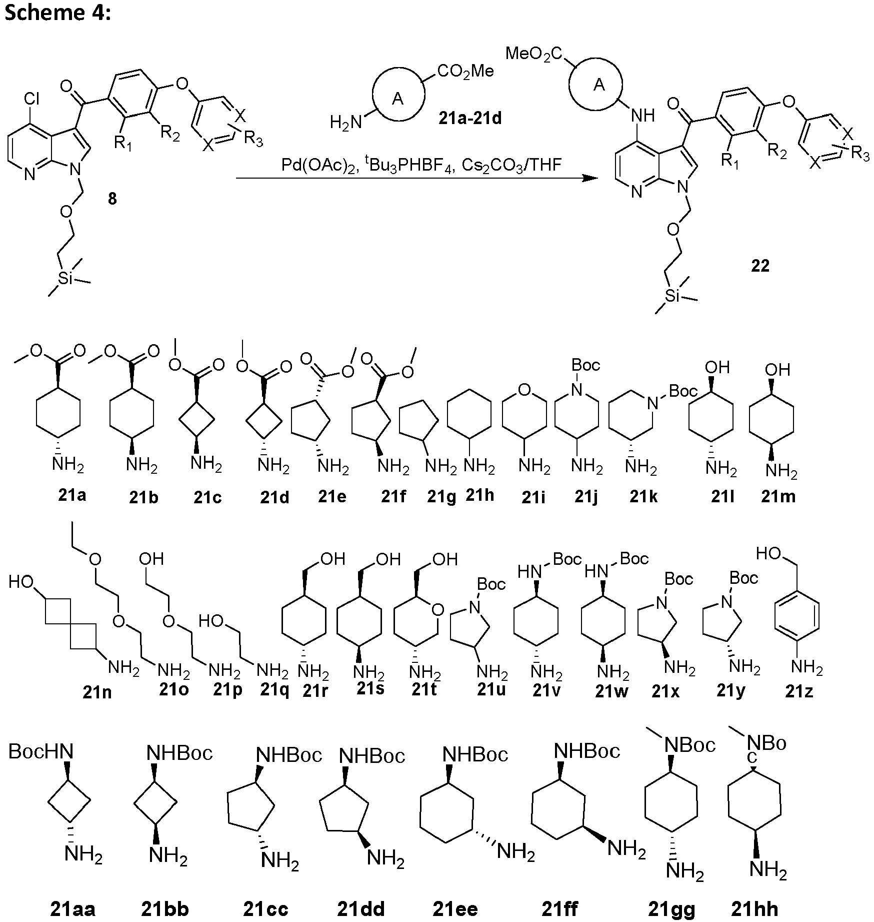

A mixture of Compound **8** (0.39 mmol) and Compound **21** (0.59 mmol), Pd(OAc)₂ (18 mg, 0.078mmol) or Pd₂(dba)₃ (71 mg, 0.078 mmol), Cs₂CO₃ (509 mg, 1.56 mmol), and ^{t}Bu₃PHBF₄(23 mg, 0.078 mmol) in THF (5 mL) or Tol (5 mL) was stirred at 100-120°C for 4-8 h, until TLC showed that Compound **8** was completely consumed. Then, the reaction mixture was cooled to room temperature, and extracted with ethyl acetate (15 mL x 3). The organic phase was washed with water and brine, and the organic layers were combined, dried over anhydrous Na₂SO₄ and evaporated to obtain a crude product. The crude product was purified by flash chromatography on silica gel using a mixture of PE and EA an eluant, to obtain a product **22** of moderate to good yield.

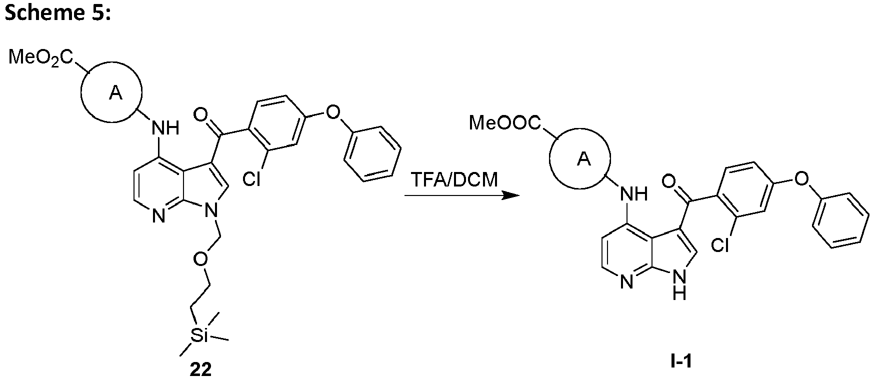

TFA (0.5 ml) was added to a solution of Compound **22** (80 mg) in DCM (1 ml). The reaction mixture was stirred at room temperature for 3-8 hrs, until TLC showed that Compound **22** was completely consumed. The solvent was removed by evaporation, and the residue was adjusted to pH 7 with saturated NaHCO₃, and extracted with ethyl acetate (8 mL x 3). The organic phase was respectively washed with water and brine, and the organic layers were combined, dried over anhydrous Na₂SO₄ and evaporated to obtain a crude product. The crude product was purified by preparative TLC, to obtain a product of moderate to good yield.

### Example 1: Methyl (1r, 4r)-4-((3-(2-chloro-4-phenoxybenzoyl)-1H-pyrrolo[2,3-b]pyridin-4-yl)amino)cyclohexane-1-carboxylate (I-1)

The compound was prepared following the general Scheme 4 with Intermediate **8a** (Table 1) and methyl (1r, 4r)-4-aminocyclohexane-1-carboxylate **(21a)** to obtain a desired compound. MS m/z: 504.2(M+H)⁺.

### Example 2: Methyl (1s, 4s)-4-((3-(2-chloro-4-phenoxybenzoyl)-1H-pyrrolo[2,3-b]pyridin-4-yl)amino)cyclohexane-1-carboxylate (I-2)

The compound was prepared following the general Scheme **4** with Intermediate **8a** (Table 1) and methyl (1s, 4s)-4-aminocyclohexane-1-carboxylate (**21b**) to obtain a desired compound. MS m/z: 504.2 (M+H)⁺.

### Example 3: Methyl (1s, 3s)-3-((3-(2-chloro-4-phenoxybenzoyl)-1H-pyrrolo[2,3-b]pyridin-4-yl)amino)cyclobutane-1-carboxylate (I-3)

The compound was prepared following the general Scheme **4** with Intermediate **8a** (Table 1) and methyl (1s, 3s)-3-aminocyclobutane-1-carboxylate (**21c**) to obtain a desired compound. MS m/z: 476.1 (M+H)⁺; ¹H NMR (DMSO-d6, 400 MHz): δ = 8.70 (d, 1H), 7.93 (d, 1H), 7.56-7.54 (m, 2H), 7.49-7.45 (m, 2H), 7.27-7.23 (m, 1H), 7.19-7.17 (m, 3H), 7.03-7.01 (m, 1H), 6.23 (d, 1H), 4.06-4.04 (m, 1H), 3.62 (s, 3H), 3.01-2.98 (m, 1H), 2.78-2.76 (m, 2H), 2.11-2.08 (m, 2H). **Example 4:** Methyl (1r, 3r)-3-((3-(2-chloro-4-phenoxybenzoyl)-1H-pyrrolo[2,3-b]pyridin-4-yl)amino)cyclobutane-1-carboxylate (**I-4**)

The compound was prepared following the general Scheme **4** with Intermediate **8a** (Table 1) and methyl (1r, 3r)-3-aminocyclobutane-1-carboxylate (**21d**) to obtain a desired compound. MS m/z: 476.1 (M+H)⁺.

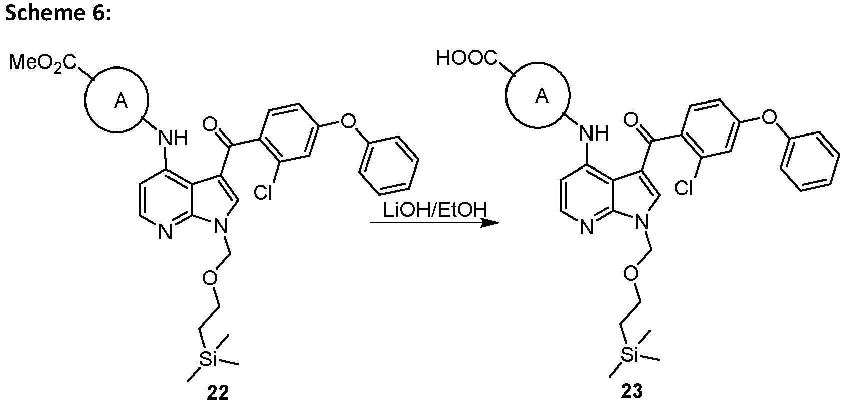

LiOH (7.6 mg, 0.316 mmol) was added to a mixed solution of Compound **22a** (100 mg, 0.158 mmol) in EtOH (2 ml) and H₂O (0.5 ml). The reaction mixture was stirred at 60-80°C for 1-3 hrs, until TLC showed that Compound **22a** was completely consumed. Then, the reaction mixture was cooled to room temperature, and evaporated to remove most of the solvent. The system was adjusted to about pH 5 with 1N HCl, and extracted with ethyl acetate (15 mL x 3). The organic phase was washed with water and brine, and the organic layers were combined, dried over anhydrous Na₂SO₄ and evaporated to obtain a crude product. The crude product was purified by preparative TLC, to obtain a product of moderate to good yield.

### Example 5: (1r, 4r)-4-((3-(2-chloro-4-phenoxybenzoyl)-1H-pyrrolo[2,3-b]pyridin-4-yl)amino)cyclohexane-1-carboxylic acid (I-5)

The compound was prepared following the general Scheme **4** with Intermediate **8a** (Table 1) and methyl (1r, 4r)-4-aminocyclohexane-1-carboxylate **(21a)** to obtain a desired compound.

MS m/z: 490.2 (M+H)⁺; ¹H NMR (DMSO-d6, 400 MHz): δ = 8.74 (d, 1H), 8.02 (d, 1H), 7.65-7.55(m, 4H), 7.37-7.29 (m, 1H), 7.29-7.26 (m, 3H), 7.12-7.10 (m, 1H), 6.48 (d, 1H), 2.61-2.45 (m, 1H), 2.24-2.20 (m, 2H), 2.10-2.06 (m, 3H), 1.66-1.63 (m, 2H), 1.45-1.42 (m, 2H); 13C NMR (DMSO, 400 MHz): δ = 189.7, 176.9, 158.8, 155.8, 150.3, 149.2, 145.9, 137.9, 134.6, 131.6, 131.1, 130.8, 125.1, 120.1, 119.5, 117.6, 116.7, 105.7, 98.8, 55.4, 50.1, 41.9, 31.7, 27.7.

### Example 6: (1s, 4s)-4-((3-(2-chloro-4-phenoxybenzoyl)-1H-pyrrolo[2,3-b]pyridin-4-yl)amino)cyclohexane-1-carboxylic acid (I-6)

The compound was prepared following the general Scheme **4** with Intermediate **8a** (Table 1) and methyl (1s, 4s)-4-aminocyclohexane-1-carboxylate **(21b)** to obtain a desired compound. MS m/z: 490.2 (M+H)⁺; ¹H NMR (DMSO-d6, 400 MHz): δ = 8.87 (d, 1H), 7.93 (d, 1H), 7.57-7.54 (m, 2H), 7.49-7.45(m, 2H), 7.26-7.23 (m, 1H), 7.19-7.17 (m, 3H), 7.03-7.01 (m, 1H), 6.38 (d, 1H), 3.78-3.73 (m, 1H), 2.47-2.43 (m, 1H), 1.89-1.72 (m, 8H).

### Example 7: (1r, 4r)-4-((3-(4-phenoxybenzoyl)-1H-pyrrolo[2,3-b]pyridin-4-yl)amino)cyclohexane-1-carboxylic acid (I-7)

The compound was prepared following the general Scheme **4** with Intermediate **8b** (Table 1) and methyl (1r, 4r)-4-aminocyclohexane-1-carboxylate **(21a)** to obtain a desired compound. MS m/z: 456.2 (M+H)⁺.

### Example 8: (1s, 4s)-4-((3-(4-phenoxybenzoyl)-1H-pyrrolo[2,3-b]pyridin-4-yl)amino)cyclohexane-1-carboxylic acid (I-8)

The compound was prepared following general Scheme **4** with Intermediate **8b** (Table 1) and methyl (1s, 4s)-4-aminocyclohexane-1-carboxylate **(21b)** to obtain a desired compound.

MS m/z: 456.6 (M+H)⁺; ¹H NMR (DMSO-d6, 400 MHz): δ =8.91 (d, 1H), 7.89 (d, 1H), 7.75 (d, 2H), 7.69 (s, 1H), 7.43-7.39(m, 2H), 7.20-7.19 (m, 1H), 7.10 (d, 2H), 7.02 (d, 2H), 6.34 (d, 1H), 3.73-3.71 (m, 1H), 2.44-2.38 (m, 1H), 1.82-1.76 (m, 4H), 1.74-1.66 (m, 4H).

### Example 9: (1S, 3R)-3-((3-(2-chloro-4-phenoxybenzoyl)-1H-pyrrolo[2,3-b]pyridin-4-yl)amino)cyclopentane-1-carboxylic acid (I-9)

The compound was prepared following the general Scheme **4** with Intermediate **8a** (Table 1) and methyl (1S, 3R)-3-aminocyclopentane-1-carboxylate (**21e**) to obtain a desired compound. MS m/z: 476.1 (M+H)⁺.

### Example 10: (1R, 3S)-3-((3-(2-chloro-4-phenoxybenzoyl)-1H-pyrrolo[2,3-b]pyridin-4-yl)amino)cyclopentane-1-carboxylic acid (I-10)

The compound was prepared following the general Scheme **4** with Intermediate **8a** (Table 1) and methyl (1R, 3S)-3-aminocyclopentane-1-carboxylate (**21f**) to obtain a desired compound. MS m/z: 476.1 (M+H)⁺.

### Example 11: (1s, 3s)-3-((3-(2-chloro-4-phenoxybenzoyl)-1H-pyrrolo[2,3-b]pyridin-4-yl)amino)cyclobutane-1-carboxylic acid (I-11)

The compound was prepared following the general Scheme **4** with Intermediate **8a** (Table 1) and methyl (1s, 3s)-3-aminocyclobutane-1-carboxylate (**21c**) to obtain a desired compound. MS m/z: 462.4 (M+H)⁺; ¹H NMR (DMSO-d6, 400 MHz): δ = 8.70 (d, 1H), 7.94 (d, 1H), 7.56-7.54 (m, 2H), 7.49-7.45 (m, 2H), 7.27-7.23 (m, 1H), 7.19-7.17 (m, 3H), 7.03-7.01 (m, 1H), 6.23 (d, 1H), 4.03-4.00 (m, 1H), 2.92-2.88 (m, 1H), 2.75-2.73 (m, 2H), 2.08-2.06 (m, 2H).

### Example 12: (1r, 3r)-3-((3-(2-chloro-4-phenoxybenzoyl)-1H-pyrrolo[2,3-b]pyridin-4-yl)amino)cyclobutane-1-carboxylic acid (I-12)

The compound was prepared following the general Scheme **4** with Intermediate **8a** (Table 1) and methyl (1r, 3r)-3-aminocyclobutane-1-carboxylate (**21d**) to obtain a desired compound. MS m/z: 462.1 (M+H)⁺.

### Example 13: (1r, 4r)-4-((3-(2-chloro-4-(pyridin-3-oxy)benzoyl)-1H-pyrrolo[2,3-b]pyridin-4-yl)amino)cyclohexane-1-carboxylic acid (I-13)

The compound was prepared following the general Scheme **4** with Intermediate **8c** (Table 1) and methyl (1r, 4r)-4-aminocyclohexane-1-carboxylate (**21a**) to obtain a desired compound. MS m/z: 491.1 (M+H)⁺.

### Example 14: (1s, 4s)-4-((3-(2-chloro-4-(pyridin-4-oxy)benzoyl)-1H-pyrrolo[2,3-b]pyridin-4-yl)amino)cyclohexane-1-carboxylic acid (I-14)

Following general Scheme **4,** the compound was prepared with Intermediate **8d** (Table 1) and methyl (1s, 4s)-4-aminocyclohexane-1-carboxylate (**21b**) to obtain a desired compound.

MS m/z: 491.1 (M+H)⁺.

### Example 15: (1r, 4r)-4-((3-(3-chloro-4-phenoxybenzoyl)-1H-pyrrolo[2,3-b]pyridin-4-yl)amino)cyclohexane-1-carboxylic acid (I-15)

The compound was prepared following the general Scheme **4** with Intermediate **8e** (Table 1) and methyl (1r, 4r)-4-aminocyclohexane-1-carboxylate **(21a)** to obtain a desired compound. MS m/z: 490.2 (M+H)⁺.

### Example 16: (1r, 4r)-4-((3-(4-phenoxy-2-(trifluoromethyl)benzoyl)-1H-pyrrolo[2,3-b]pyridin-4-yl)amino)cyclohexane-1-carboxylic acid (I-16)

The compound was prepared following the general Scheme **4** with Intermediate **8f** (Table 1) and methyl (1r, 4r)-4-aminocyclohexane-1-carboxylate **(21a)** to obtain a desired compound. MS m/z: 524.2 (M+H)⁺.

### Example 17: (1s, 4s)-4-((3-(4-phenoxy-2-(trifluoromethyl)benzoyl)-1H-pyrrolo[2,3-b]pyridin-4-yl)amino)cyclohexane-1-carboxylic acid (I-17)

Following general Scheme **4,** the compound was prepared with Intermediate **8f** (Table 1) and methyl (1s, 4s)-4-aminocyclohexane-1-carboxylate **(21b)** to obtain a desired compound.

MS m/z: 524.2 (M+H)⁺.

### Example 18: (1r, 4r)-4-((3-(2-methyl-4-phenoxybenzoyl)-1H-pyrrolo[2,3-b]pyridin-4-yl)amino)cyclohexane-1-carboxylic acid (I-18)

The compound was prepared following the general Scheme **4** with Intermediate **8 g** (Table 1) and methyl (1r, 4r)-4-aminocyclohexane-1-carboxylate **(21a),** to obtain a desired compound. MS m/z: 470.2 (M+H)⁺.

### Example 19: (2-chloro-4-phenoxyphenyl)(4-(cyclopentylamino)-1H-pyrrolo[2,3-b]pyridin-3-yl)methanone (I-19)

The compound was prepared following the general Scheme **4** with Intermediate **8a** (Table 1) and cyclopentanamine (**21g**), to obtain a desired product.

MS m/z: 432.1 (M+H)⁺.

### Example 20: (2-chloro-4-phenoxyphenyl)(4-(cyclohexylamino)-1H-pyrrolo[2,3-b]pyridin-3-yl)methanone (I-20)

The compound was prepared following the general Scheme **4** with Intermediate **8a** (Table 1) and cyclohexanamine **(21h)** to obtain a desired product.

MS m/z: 446.2 (M+H)⁺.

### Example 21: (2-chloro-4-phenoxyphenyl)(4-((tetrahydro-2H-pyran-4-yl)amino)-1H-pyrrolo[2,3-b]pyridin-3-yl)methanone (I-21)

The compound was prepared following the general Scheme **4** with Intermediate **8a** (Table 1) and tetrahydro-2H-pyran-4-amine (**21i**) to obtain a desired product.

MS m/z: 448.1 (M+H)⁺.

### Example 22: (2-chloro-4-phenoxyphenyl)(4-(piperidin-4-ylamino)-1H-pyrrolo[2,3-b]pyridin-3-yl)methanone (I-22)

The compound was prepared following the general Scheme **4** with Intermediate **8a** (Table 1) and t-butyl 4-aminopiperidin-1-carboxylate (**21j**) to obtain a desired compound.

MS m/z: 447.2 (M+H)⁺; ¹H NMR (DMSO-d6, 400 MHz): δ =8.75 (d, 1H), 8.06 (d, 1H), 7.66 (s, 1H), 7.60-7.56(m, 3H), 7.37-7.33 (m, 1H), 7.29-7.26 (m, 3H), 7.14-7.11 (m, 1H), 6.53 (d, 1H), 3.96-3.94 (m, 1H), 3.46-3.42 (m, 2H), 3.24-3.19 (m, 2H), 2.34-2.30 (m, 2H), 1.85-1.80 (m, 2H). **Example 23:** (R)-(2-chloro-4-phenoxyphenyl)(4-(piperidin-3-ylamino)-1H-pyrrolo[2,3-b]pyridin-3-yl)methanone (**I-23**)

The compound was prepared following the general Scheme **4** with Intermediate **8a** (Table 1) and t-butyl (R)-3-aminopyridin-1-carboxylate (**21k**), to obtain a desired product.

MS m/z: 447.2 (M+H)⁺; ¹H NMR (DMSO-d6, 400 MHz): δ =9.00 (s, 1H), 8.60 (d, 1H), 7.99 (d, 1H), 7.54-7.46(m, 4H), 7.27-7.23 (m, 1H), 7.19-7.17 (m, 3H), 7.04-7.01 (m, 1H), 6.46 (d, 1H), 3.96-3.94 (m, 1H), 3.27-3.24 (m, 2H), 2.97-2.92 (m, 1H), 2.86-2.81 (m, 1H), 2.16-2.13 (m, 1H), 1.98-1.95 (m, 1H), 1.87-1.83 (m, 1H), 1.72-1.67 (m, 1H).

### Example 24: (2-chloro-4-phenoxyphenyl)(4-(((1r, 4r)-4-hydroxycyclohexyl)amino)-1H-pyrrolo[2,3-b]pyridin-3-yl)methanone(I-24)

The compound was prepared following the general Scheme **4** with Intermediate **8a** (Table 1) and (1r, 4r)-4-aminocyclohexane-1-ol (**21l**) to obtain a desired product.

MS m/z: 462.2 (M+H)⁺; ¹H NMR (DMSO-d6, 400 MHz): δ =8.52 (d, 1H), 7.90 (d, 1H), 7.53 (d, 1H), 7.50-7.46(m, 4H), 7.27-7.23 (m, 1H), 7.19-7.16 (m, 3H), 7.03-7.00 (m, 1H), 6.33 (d, 1H), 4.61-4.60 (m, 1H), 3.54-3.53 (m, 1H), 2.09-2.06 (m, 2H), 1.90-1.87 (m, 2H), 1.39-1.32 (m, 4H). **Example 25:** (2-chloro-4-phenoxyphenyl)(4-(((1s, 4s)-4-hydroxycyclohexyl)amino)-1H-pyrrolo[2,3-b]pyridin-3-yl)methanone (**I-25**)

The compound was prepared following the general Scheme **4** with Intermediate **8a** (Table 1) and (1s, 4s)-4-aminocyclohexane-1-ol (**21m**) to obtain a desired product.

MS m/z: 462.2 (M+H)⁺; ¹H NMR (DMSO-d6, 400 MHz): δ =7.77 (d, 1H), 7.44-7.40(m, 3H), 7.24-7.20 (m, 2H), 7.13-7.11 (m, 2H), 6.78-6.77 (m, 1H), 6.63-6.61 (m, 1H), 6.34 (d, 1H), 5.61-5.59 (m, 1H), 5.13-5.10 (m, 1H), 3.66-3.63 (m, 1H), 1.86-1.84 (m, 4H), 1.56-1.54 (m, 4H).

### Example 26: (2-chloro-4-phenoxyphenyl)(4-((6-hydroxyspiro[3.3]heptan-2-yl)amino)-1H-pyrrolo[2,3-b]pyridin-3-yl)methanone (I-26)

The compound was prepared following the general Scheme **4** with Intermediate **8a** (Table 1) and 6-aminospiro[3.3]heptan-2-ol (**21n**) to obtain a desired product.

MS m/z: 474.2 (M+H)⁺.

### Example 27: (2-chloro-4-phenoxyphenyl)(4-((2-(2-ethoxyethoxy)ethyl)amino)-1H-pyrrolo[2,3-b]pyridin-3-yl)methanone (I-27)

The compound was prepared following the general Scheme **4** with Intermediate **8a** (Table 1) and 2-(2-ethoxyethoxy)ethan-1-amine (**21o**) to obtain a desired product

MS m/z: 480.2 (M+H)⁺.

### Example 28: (2-chloro-4-phenoxyphenyl)(4-((2-(2-hydroxyethoxy)ethyl)amino)-1H-pyrrolo[2,3-b]pyridin-3-yl)methanone (I-28)

The compound was prepared following the general Scheme **4** with Intermediate **8a** (Table 1) and 2-(2-aminoethoxy)ethan-1-ol (**21p**) to obtain a desired product.

MS m/z: 452.1 (M+H)⁺; ¹H NMR (DMSO-d6, 400 MHz): δ =8.65-8.63 (m, 1H), 7.94 (d, 1H), 7.54-7.45(m, 4H), 7.28-7.24 (m, 1H), 7.19-7.17 (m, 3H), 7.03-7.01 (m, 1H), 6.33 (d, 1H), 4.57-4.54 (m, 1H), 3.72-3.70 (m, 2H), 3.55-3.51 (m, 4H), 3.45-3.41 (m, 2H).

### Example 29: (2-chloro-4-phenoxyphenyl)(4-((2-hydroxyethyl)amino)-1H-pyrrolo[2,3-b]pyridin-3-yl)methanone (I-29)

The compound was prepared following the general Scheme **4** with Intermediate **8a** (Table 1) and 2-aminoethan-1-ol (**21q**) to obtain a desired product.

MS m/z: 408.1 (M+H)⁺.

### Example 30: (2-chloro-4-phenoxyphenyl)(4-(((1r, 4r)-4-(hydroxymethyl)cyclohexyl)amino)-1H-pyrrolo[2,3-b]pyridin-3-yl)methanone(I-30)

The compound was prepared following the general Scheme 4 with Intermediate **8a** (Table 1) and ((1r, 4r)-4-aminocyclohexyl)methanol (**21r**) to obtain a desired product.

MS m/z: 476.2 (M+H)⁺; ¹H NMR (DMSO-d6, 400 MHz): δ =9.08 (d, 1H), 7.98 (d, 1H), 7.61 (s, 1H), 7.57 (d, 1H), 7.51-7.47(m, 2H), 7.28-7.25 (m, 1H), 7.20-7.18 (m, 3H), 7.05-7.02 (m, 1H), 6.53 (d, 1H), 4.45-4.43 (m, 1H), 3.33-3.28 (m, 2H), 2.15-2.13 (m, 2H), 1.85-1.82 (m, 2H), 1.45-1.43 (m, 1H), 1.33-1.30 (m, 2H), 1.18-1.14 (m, 2H).

### Example 31: (2-chloro-4-phenoxyphenyl)(4-(((1s, 4s)-4-(hydroxymethyl)cyclohexyl)amino)-1H-pyrrolo[2,3-b]pyridin-3-yl)methanone(I-31)

The compound was prepared following the general Scheme **4** with Intermediate **8a** (Table 1) and ((1s, 4s)-4-aminocyclohexyl)methanol (**21s**) to obtain a desired product.

MS m/z: 477.2 (M+H)⁺; ¹H NMR (DMSO-d6, 400 MHz): δ =8.88 (d, 1H), 7.92 (d, 1H), 7.57-7.53 (m, 2H), 7.48-7.46(m, 2H), 7.31-7.26 (m, 1H), 7.19-7.17 (m, 3H), 7.04-7.02 (m, 1H), 6.84 (d, 1H), 4.46-4.45 (m, 1H), 3.88-3.87 (m, 1H), 3.29-3.27 (m, 2H), 1.80-1.76 (m, 2H), 1.65-1.63 (m, 2H), 1.55-1.52 (m, 2H), 1.38-1.32 (m, 2H).

### Example 32: (2-chloro-4-phenoxyphenyl)(4-(((3R, 6S)-6-(hydroxymethyl)tetrahydro-2H-pyran-3-yl)amino)-1H-pyrrolo[2,3-b]pyridin-3-yl)methanone (I-32)

The compound was prepared following the general Scheme **4** with Intermediate **8a** (Table 1) and ((2S, 5R)-5-aminotetrahydro-2H-pyran-2-yl)methanol (**21t**) to obtain a desired product.

MS m/z: 478.2 (M+H)⁺; ¹H NMR (DMSO-d6, 400 MHz): δ =8.46 (d, 1H), 7.93 (d, 1H), 7.55-7.53(m, 2H), 7.50-7.46 (m, 2H), 7.27-7.23 (m, 1H), 7.20-7.17 (m, 3H), 7.03-7.00 (m, 1H), 6.42 (d, 1H), 4.70 (s, 1H), 4.13-4.10 (m, 1H), 3.61-3.57 (m, 1H), 3.38-3.37 (m, 1H), 3.15-3.10(m, 2H), 2.25-2.21 (m, 1H), 1.80-1.76 (m, 1H), 1.55-1.52 (m, 1H), 1.23-1.15 (m, 1H), 1.18-1.14 (m, 2H).

### Example 33: (4-(((1s, 4s)-4-(hydroxymethyl)cyclohexyl)amino)-1H-pyrrolo[2,3-b]pyridin-3-yl)(4-phenoxyphenyl)methanone (I-33)

The compound was prepared following the general Scheme **4** with Intermediate **8b** (Table 1) and ((1s, 4s)-4-aminocyclohexyl)methanol (**21s**) to obtain a desired product.

MS m/z: 442.2 (M+H)⁺.

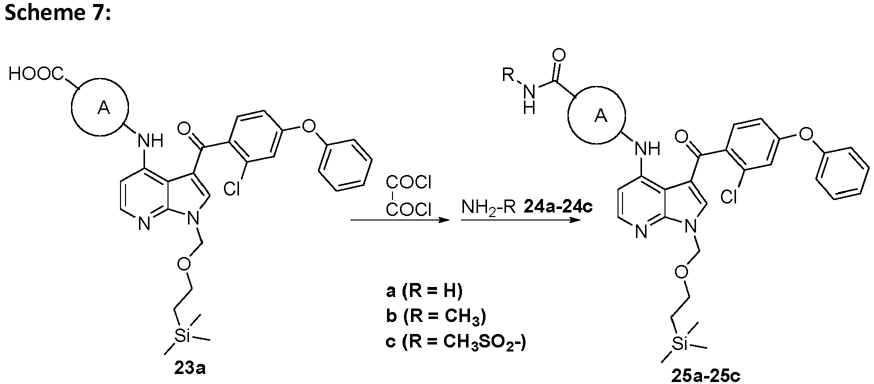

Three drops of DMF was added to a mixed solution of Compound **23a** (150 mg, 0.24 mmol) in THF (3 ml), and the reaction mixture was cooled to 0°C, and then oxalyl chloride (34 mg, 0.27 mmol) was added dropwise. The reaction mixture was stirred at 0°C for 1-2 hrs, until TLC showed that Compound **23a** was completely consumed. The solvent was removed by evaporation, and the residue was taken up in THF, and respectively added to a solution of **24a-c**(0.73 mmol) in THF (3 ml). The reaction mixture was continuously stirred for 0.5-1 hrs, and extracted with ethyl acetate (15 mL x 3). The organic phase was separately washed with water and brine, and the organic layers were combined, dried over anhydrous Na₂SO₄, and evaporated to remove the solvent, so as to obtain a crude product. The crude product was purified by preparative TLC to obtain products **25a-c** of moderate to good yield.

### Example 34: (1r, 4r)-4-((3-(2-chloro-4-phenoxybenzoyl)-1H-pyrrolo[2,3-b]pyridin-4-yl)amino)cyclohexane-1-carboxamide (I-34)

The compound was prepared following the general Scheme **7** with Intermediate **23a** and aqueous ammonia (**24a**) to obtain a desired product.

MS m/z: 489.2 (M+H)⁺.

### Example 35: (1r, 4r)-4-((3-(2-chloro-4-phenoxybenzoyl)-1H-pyrrolo[2,3-b]pyridin-4-yl)amino)-N-methylcyclohexane-1-carboxamide (I-35)

The compound was prepared following the general Scheme **7** with Intermediate **23a** and methylamine (**24b**) to obtain a desired product.

MS m/z: 503.2 (M+H)⁺; ¹H NMR (DMSO-d6, 400 MHz): δ =8.66 (d, 1H), 7.93 (d, 1H), 7.73-7.71 (m, 1H), 7.55-7.52(m, 2H), 7.49-7.46 (m, 2H), 7.27-7.23 (m, 1H), 7.19-7.17 (m, 3H), 7.03-7.00 (m, 1H), 6.41 (d, 1H), 3.49-3.46 (m, 1H), 2.58 (d, 3H), 2.20-2.14 (m, 3H), 1.83-1.80 (m, 2H), 1.63-1.53 (m, 2H), 1.33-1.22 (m, 2H); 13C NMR (DMSO, 400 MHz): δ = 189.8, 175.6, 158.8, 155.8, 150.3, 149.3, 134.6, 131.6, 131.1, 130.8, 125.1, 120.1, 119.5, 117.6, 116.7, 105.8, 98.8, 55.4, 50.3, 43.6, 32.1, 28.4, 25.9.

### Example 36: (1r, 4r)-4-((3-(2-chloro-4-phenoxybenzoyl)-1H-pyrrolo[2,3-b]pyridin-4-yl)amino)-N-(methanesulfonyl)cyclohexane-1-carboxamide (I-36)

The compound was prepared following the general Scheme **7** with Intermediate **23a** and methylsulfonamide (**24c**) to obtain a desired product.

MS m/z: 567.1 (M+H)⁺.

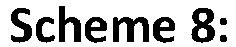

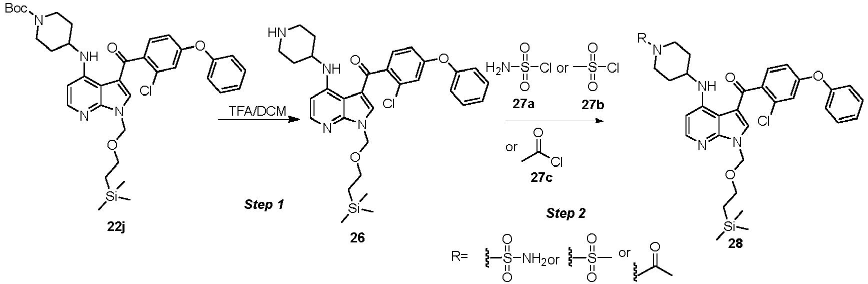

Step 1: TFA (0.5 ml) was added to a solution of Compound **22j** (200 mg) in DCM (5 ml). The reaction mixture was stirred at room temperature for 0.5-1 hrs, until TLC showed that Compound **22j** was completely consumed. The system was evaporated to remove most of the solvent, adjusted to pH 7 with saturated NaHCO₃, and extracted with ethyl acetate (15 mL x 3). The organic phase was washed with water and brine, and the organic phases were combined, dried over anhydrous Na₂SO₄ and evaporated to obtain a crude compound. The crude product was purified by preparative TLC, to obtain a product of moderate to good yield.

Step 2: DIPEA (67 mg, 0.52 mmol) was added to a mixed solution of Compound **26** (100 mg, 0.17 mmol) in DCM (3 ml). The reaction mixture was cooled to 0°C, and then Compound **27a** (30 mg, 0.26 mmol) was added batchwise. The reaction mixture was stirred at 0°C to room temperature for 1-2 hrs, until TLC showed that Compound **26** was completely consumed. The system was evaporated to remove most of the solvent and extracted with ethyl acetate (15 mL x 3). The organic phase was washed with water and brine, and the organic layers were combined, dried over anhydrous Na₂SO₄, and evaporated to remove the solvent, so as to obtain a crude product. The crude product was purified by preparative TLC, to obtain the product **28** (80 mg, yield 70%).

### Example 37: 4-((3-(2-chloro-4-phenoxybenzoyl)-1H-pyrrolo[2,3-b]pyridin-4-yl)amino)piperidin-1-sulfonamide (I-37)

The compound was prepared following the general Scheme **8** with Intermediate **22j** and aminosulfonyl chloride (**27a**) to obtain a desired product.

MS m/z: 526.1 (M+H)⁺; ¹H NMR (DMSO-d6, 400 MHz): δ =8.62 (d, 1H), 7.94 (d, 1H), 7.55-7.53(m, 2H), 7.50-7.46 (m, 2H), 7.27-7.23 (m, 1H), 7.19-7.17 (m, 3H), 7.03-7.01 (m, 1H), 6.82 (s, 2H), 6.41 (d, 1H), 3.65-3.63 (m, 1H), 3.47-3.44 (m, 2H), 2.90-2.85 (m, 2H), 2.14-2.12 (m, 2H), 1.63-1.54 (m, 2H).

### Example 38: (2-chloro-4-phenoxyphenyl)(4-((1-(methanesulfonyl)piperidin-4-yl)amino)-1H-pyrrolo[2,3-b]pyridin-3-yl)methanone (I-38)

The compound was prepared following the general Scheme **8** with Intermediate **22j** and methansulfonyl chloride (**27b**) to obtain a desired product.

MS m/z: 525.1 (M+H)⁺; ¹H NMR (DMSO-d6, 400 MHz): δ =8.69 (d, 1H), 7.95 (d, 1H), 7.56-7.54(m, 2H), 7.50-7.46 (m, 2H), 7.27-7.23 (m, 1H), 7.20-7.18 (m, 3H), 7.05-7.01 (m, 1H), 6.41 (d, 1H), 3.75-3.73 (m, 1H), 3.54-3.52 (m, 2H), 3.10-3.05 (m, 2H), 2.91 (s, 3H), 2.14-2.11 (m, 2H), 1.64-1.59 (m, 2H).

### Example 39: 3-((3-(2-chloro-4-phenoxybenzoyl)-1H-pyrrolo[2,3-b]pyridin-4-yl)amino)cyclopentane-1-sulfonamide (I-39)

The compound was prepared following the general Scheme **8** with Intermediate **22u** and aminosulfonyl chloride (**27a**) to obtain a desired product.

MS m/z: 511.1 (M+H)⁺.

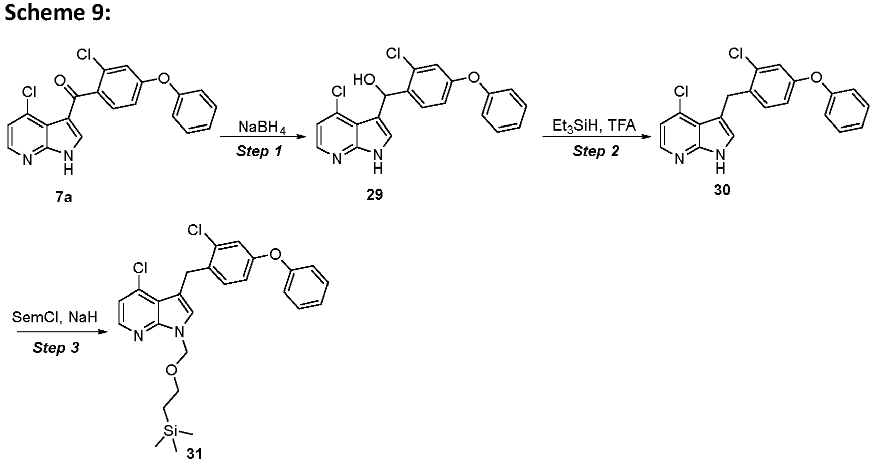

Step 1: Under N₂ atmosphere, NaBH₄ (498mg, 13.1mmol) was added batchwise to a mixed solution of Compound **7a** (0.5 g, 1.31 mmol) in EtOH (15 mL). The reaction mixture was stirred overnight at room temperature, until TLC showed that Compound **7a** was completely consumed. The reaction solution was quenched with saturated NH₄Cl, and extracted with ethyl acetate (20 mL x 3). The organic phase was washed separately with water and brine. The organic phases were combined, dried over Na₂SO₄ and evaporated to obtain a crude product **29** (500 mg, yield: 100%). The crude product was used directly in the next step of synthesis without further purification.

Step 2: TFA (594 mg, 5.21 mmol) and Et₃SiH (606 mg, 5.21 mmol) were added to a mixed solution of Compound **29** (500 mg, 1.30 mmol) in DCM (8 ml). The reaction mixture was stirred at room temperature until TLC showed that Compound **29** was completely consumed. Most of the solvent was removed by evaporation, and the organic phase was adjusted to pH 7 with saturated NaHCO₃, and extracted with ethyl acetate (15 mL x 3). The organic phase was washed separately with water and brine, dried over Na₂SO₄ and evaporated to obtain a crude product, which was purified by column chromatography on silica gel to obtain Compound **30** (300 mg, yield: 63%).

Step 3: Under N₂ atmosphere, the solution of Compound **30** (300 mg, 0.815 mmol) in THF (8 mL) was cooled to about 0°C. NaH (49 mg, 1.22 mmol, 60% in oil) was added batchwise to the solution, and then the reaction mixture was stirred for 30 min. SEMCI (203 mg, 1.22 mmol) was added dropwise, and then the system was stirred at room temperature for 1-3 hrs, until TLC showed that Compound **30** was completely consumed. The reaction was quenched with saturated NH₄Cl at 0°C, and extracted with ethyl acetate (10 ml x 3). The combined organic layer was washed with water and brine, dried over Na₂SO₄ and evaporated to obtain a crude product, which was purified by column chromatography on silica gel to obtain Compound **31** (360 mg, yield: 89%).

### Example 40: (1r, 4r)-4-((3-(2-chloro-4-phenoxybenzyl)-1H-pyrrolo[2,3-b]pyridin-4-yl)amino)cyclohexane-1-carboxylic acid (I-40)

The compound was prepared following the general Scheme **9** with Intermediate **31** and methyl (1r, 4r)-4-aminocyclohexane-1-carboxylate (**21a**) to obtain a desired product.

MS m/z: 476.5 (M+H)⁺; ¹H NMR (DMSO-d6, 400 MHz): δ =11.41 (s, 1H), 7.87 (d, 1H), 7.43-7.39(m, 2H), 7.20-7.16 (m, 2H), 7.05-7.01 (m, 3H), 6.94-6.92 (m, 1H), 6.90 (s, 1H), 6.34 (d, 1H), 4.91-4.89 (m, 1H), 4.25 (s, 2H), 2.13-2.07 (m, 1H), 1.89-1.85 (m, 4H), 1.50-1.42 (m, 2H), 1.06-1.01 (m, 2H).

### Example 41: (1s, 4s)-4-((3-(2-chloro-4-phenoxybenzyl)-1H-pyrrolo[2,3-b]pyridin-4-yl)amino)cyclohexane-1-carboxylic acid (I-41)

The compound was prepared following general Scheme **9** with Intermediate **31** and methyl (1s, 4s)-4-aminocyclohexane-1-carboxylate (**21b**) to obtain a desired product.

MS m/z: 476.2 (M+H)⁺; ¹H NMR (DMSO-d6, 400 MHz): δ =11.61 (s, 1H), 7.89 (d, 1H), 7.42-7.38(m, 2H), 7.19-7.15 (m, 1H), 7.14-7.13 (m, 1H), 7.04-7.02 (m, 2H), 6.99-6.96 (m, 1H), 6.91 (s, 1H), 6.88-6.85 (m, 1H), 6.37 (d, 1H), 5.24-5.22 (m, 1H), 4.26 (s, 2H), 3.59-3.57 (m, 1H), 2.42-2.41 (m, 1H), 1.64-1.60 (m, 6H), 1.45-1.41 (m, 2H).

### Example 42: ((1s, 4s)-4-((3-(2-chloro-4-phenoxybenzyl)-1H-pyrrolo[2,3-b]pyridin-4-yl)amino)cyclohexyl)methanol (I-42)

The compound was prepared following general Scheme **9** with Intermediate **33** and ((1s, 4s)-4-aminocyclohexyl) methanol **(21s)** to obtain a desired product.

MS m/z: 462.2 (M+H)⁺.

### Example 43: ((1r, 4r)-4-((3-(2-chloro-4-phenoxybenzyl)-1H-pyrrolo[2,3-b]pyridin-4-yl)amino)cyclohexyl)methanol (I-43)

The compound was prepared following general Scheme **9** with Intermediate **33** and ((1r, 4r)-4-aminocyclohexyl) methanol **(21r)** to obtain a desired product.

MS m/z: 462.2 (M+H)⁺.

### Example 44: ((1s, 4s)-4-((3-(4-phenoxybenzyl)-1H-pyrrolo[2,3-b]pyridin-4-yl)amino)cyclohexyl)methanol (I-44)

The compound was prepared following general Scheme **9** with Intermediate **33** and methyl (1s, 4s)-4-aminocyclohexane-1-carboxylate **(21b)** to obtain a desired product.

MS m/z: 428.2 (M+H)⁺.

### Example 45: (4((4(hydroxymethyl)phenyl)amino)-1H-pyrrolo[2,3-b]pyridin-3-yl )(4-phenoxyphenyl)methanone (I-45)

The compound was prepared following the general Scheme **4** with Intermediate **8a** (Table 1) and p-aminobenzyl alcohol **(21z)** to obtain a desired product.

MS m/z: 436.2 (M+H)⁺.

### Example 46: (1r, 4r)-4-((3-(2-chloro-4-(pyridin-3-oxy)benzoyl)-1H-pyrrolo[2,3-b]pyridin-4-yl)amino)cyclohexane-1-carboxylic acid (I-46)

The compound was prepared following the general Scheme **4** with Intermediate **8c** (Table 1) and methyl (1r, 4r)-4-aminocyclohexane-1-carboxylate **(21a)** to obtain a desired compound. MS m/z: 491.1 (M+H)⁺.

### Example 47: (1s, 4s)-4-((3-(2-chloro-4-(pyridin-3-oxy)benzoyl)-1H-pyrrolo[2,3-b]pyridin-4-yl)amino)cyclohexane-1-carboxylic acid (I-47)

Following general Scheme **4,** the compound was prepared with Intermediate **8c** (Table 1) and methyl (1s, 4s)-4-aminocyclohexane-1-carboxylate **(21b)** to obtain a desired compound.

MS m/z: 491.1 (M+H)⁺.

### Example 48: (1s, 4s)-4-((3-(2-chloro-4-(pyridin-4-oxy)benzoyl)-1H-pyrrolo[2,3-b]pyridin-4-yl)amino)cyclohexane-1-carboxylic acid (I-48)

Following general Scheme **4,** the compound was prepared with Intermediate **8d** (Table 1) and methyl (1s, 4s)-4-aminocyclohexane-1-carboxylate (**21b**) to obtain a desired compound.

MS m/z: 491.1 (M+H)⁺.

### Example 49: (2-chloro-4-(pyridin-3-oxy)phenyl)(4-((1r, 4r)-4-(hydroxymethyl)cyclohexyl)amino)-1H-pyrrolo[2,3-b]pyridin-3-yl)methanone (I-49)

The compound was prepared following the general Scheme **4** with Intermediate **8c** (Table 1) and ((1r, 4r)-4-aminocyclohexyl)methanol (**21r**) to obtain a desired product.

MS m/z: 477.2 (M+H)⁺.

### Example 50: (2-chloro-4-(pyridin-3-oxy)phenyl)(4-(((1s, 4s)-4-(hydroxymethyl)cyclohexyl)amino)-1H-pyrrolo[2,3-b]pyridin-3-yl)methanone (I-50)

The compound was prepared following the general Scheme **4** with Intermediate **8c** (Table 1) and ((1s, 4s)-4-aminocyclohexyl)methanol (**21s**) to obtain a desired product.

MS m/z: 477.2 (M+H)⁺.

### Example 51: (2-chloro-4-(pyridin-4-oxy)phenyl)(4-((1s, 4s)-4-(hydroxymethyl)cyclohexyl)amino)-1H-pyrrolo[2,3-b]pyridin-3-yl)methanone (I-51)

The compound was prepared following the general Scheme **4** with Intermediate **8d** (Table 1) and ((1s, 4s)-4-aminocyclohexyl)methanol (**21s**) to obtain a desired product.

MS m/z: 477.2 (M+H)⁺.

### Example 52: (2-chloro-4-((5-methoxypyridin-3-yl)oxy)phenyl)(4-(((1r, 4r)-4-(hydroxymethyl)cyclohexyl)amino)-1H-pyrrolo[2,3-b]pyridin-3-yl)methanone(I-52)

The compound was prepared following the general Scheme **4** with Intermediate **8h** (Table 1) and ((1r, 4r)-4-aminocyclohexyl)methanol (**21r**) to obtain a desired product.

MS m/z: 507.2 (M+H)⁺.

### Example 53: (2-chloro-4-((2-methoxypyridin-4-yl)oxy)phenyl)(4-(((1s, 4s)-4)-(hydroxymethyl)cyclohexyl)amino)-1H-pyrrolo[2,3-b]pyridin-3-yl)methanone (I-53)

The compound was prepared following the general Scheme **4** with Intermediate **8i** (Table 1) and ((1s, 4s)-4-aminocyclohexyl)methanol (**21s**) to obtain a desired product.

MS m/z: 507.2 (M+H)⁺.

### Example 54: (2-chloro-4-((5-methylpyridin-3-yl)oxy)phenyl)(4-((1r, 4r)-4-(hydroxymethyl)cyclohexyl)amino)-1H-pyrrolo[2,3-b]pyridin-3-yl)methanone (I-54)

The compound was prepared following the general Scheme **4** with Intermediate **8j** (Table 1) and ((1r, 4r)-4-aminocyclohexyl)methanol **(21r)** to obtain a desired product.

MS m/z: 491.2 (M+H)⁺.

### Example 55: (2-chloro-4-((5-(trifluoromethyl)pyridin-3-yl)oxy)phenyl)(4-(((1r, 4r)-4-(hydroxymethyl)cyclohexyl)amino)-1H-pyrrolo[2,3-b]pyridin-3-yl)methanone (I-55)

The compound was prepared following the general Scheme **4** with Intermediate **8k** (Table 1) and ((1r, 4r)-4-aminocyclohexyl)methanol **(21r)** to obtain a desired product.

MS m/z: 545.2 (M+H)⁺.

### Example 56: (2-chloro-4-((5-(trifluoromethyl)pyridin-3-yl)oxy)phenyl)(4-((1s, 4s)-4-(hydroxymethyl)cyclohexyl)amino)-1H-pyrrolo[2,3-b]pyridin-3-yl)methanone (I-56)

The compound was prepared following the general Scheme **4** with Intermediate **8k** (Table 1) and ((1s, 4s)-4-aminocyclohexyl)methanol **(21s)** to obtain a desired product.

MS m/z: 545.2 (M+H)⁺.

### Example 57: (2-chloro-4-(pyrimidin-5-oxy)phenyl)(4-((1r, 4r)-4-(hydroxymethyl)cyclohexyl)amino)-1H-pyrrolo[2,3-b]pyridin-3-yl)methanone (I-57)

The compound was prepared following the general Scheme **4** with Intermediate **8l** (Table 1) and ((1r, 4r)-4-aminocyclohexyl)methanol **(21r)** to obtain a desired product.

MS m/z: 478.2 (M+H)⁺.

### Example 58: N-(3-chloro-4-(4-(((1r, 4r)-4-(hydroxymethyl)cyclohexyl)amino)-1H-pyrrolo[2,3-b]pyridin-3-carbonyl)phenyl)-2-methoxybenzamide (I-58)

The compound was prepared following the general Scheme **4** with Intermediate **8o** (Table 1) and ((1r, 4r)-4-aminocyclohexyl)methanol **(21r)** to obtain a desired product.

MS m/z: 533.2 (M+H)⁺.

### Example 59: N-(3-chloro-4-(4-(((1r, 4r)-4-(hydroxymethyl)cyclohexyl)amino)-1H-pyrrolo[2,3-b]pyridin-3-carbonyl)phenyl)-5-fluoro-2-methoxybenzamide (I-59)

The compound was prepared following the general Scheme **4** with Intermediate **8p** (Table 1) and ((1r, 4r)-4-aminocyclohexyl)methanol **(21r)** to obtain a desired product.

MS m/z: 551.2 (M+H)⁺.

### Example 60: N-(3-chloro-4-(4-(((1r, 4r)-4-(hydroxymethyl)cyclohexyl)amino)-1H-pyrrolo[2,3-b]pyridin-3-carbonyl)benzyl)-5-fluoro-2-methoxybenzamide (I-60)

The compound was prepared following the general Scheme **4** with Intermediate **8q** (Table 1) and ((1r, 4r)-4-aminocyclohexyl)methanol **(21r)** to obtain a desired product.

MS m/z: 565.2 (M+H)⁺.

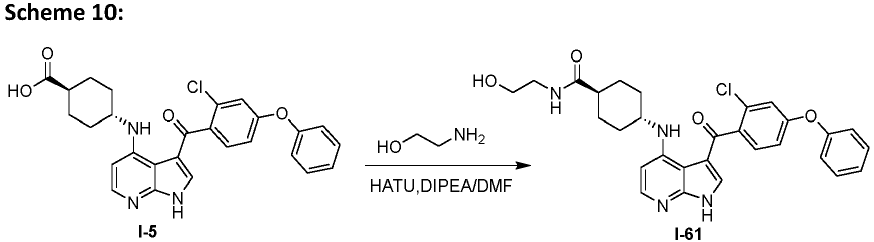

### Example 61: (1r, 4r)-4-((3-(2-chloro-4-phenoxybenzoyl)-1H-pyrrolo[2,3-b]pyridin-4-yl)amino)-N-(2-hydroxyethyl)cyclohexane-1-carboxamide (I-61)

The compound was prepared following the general Scheme **10** with Compound **I-5** and 2-aminoethan-1-ol to obtain a desired product.

MS m/z: 533.2 (M+H)⁺.

### Example 62: (1r, 4r)-4-((3-(2-chloro-4-(2-methoxyphenoxy)benzoyl)-1H-pyrrolo[2,3-b]pyridin-4-yl)amino)cyclohexane-1-carboxylic acid (I-62)

The compound was prepared following the general Scheme **4** with Intermediate **8m** (Table 1) and methyl (1r, 4r)-4-aminocyclohexane-1-carboxylate (**21a**) to obtain a desired compound. MS m/z: 520.2 (M+H)⁺.

### Example 63: (1r, 4r)-4-((3-(2-chloro-4-(5-fluoro-2-methoxyphenoxy)benzoyl)-1H-pyrrolo[2,3-b]pyridin-4-yl)amino)cyclohexane-1-carboxylic acid (I-63)

The compound was prepared following the general Scheme **4** with Intermediate **8n** (Table 1) and methyl (1r, 4r)-4-aminocyclohexane-1-carboxylate (**21a**) to obtain a desired compound. MS m/z: 538.2 (M+H)⁺.

### Example 64: (1r, 4r)-4-((3-(2-chloro-4-phenoxybenzoyl)-1H-pyrazolo[3,4-b]pyridin-4-yl)amino)cyclohexane-1-carboxylic acid (I-64)

The compound was prepared following the general Scheme **4** with Intermediate **8r** (Table 1) and methyl (1r, 4r)-4-aminocyclohexane-1-carboxylate (**21a**) to obtain a desired compound. MS m/z: 491.1 (M+H)⁺.

### Example 65: (2-chloro-4-phenoxyphenyl)(4-(((1r, 4r)-4-(hydroxymethyl)cyclohexyl)amino)-1H-pyrazolo[3,4-b]pyridin-3-yl)methanone (I-65)

The compound was prepared following the general Scheme **4** with Intermediate **8r** (Table 1) and ((1r, 4r)-4-aminocyclohexyl)methanol (**21r**) to obtain a desired product.

MS m/z: 477.2 (M+H)⁺.

### Example 66: (2-chloro-4-phenoxyphenyl)(4-(((3R, 6S)-6-(hydroxymethyl)tetrahydro-2H-pyran-3-yl)amino)-1H-pyrazolo[3,4-b]pyridin-3-yl)methanone (I-66)

The compound was prepared following the general Scheme **4** with Intermediate **8r** (Table 1) and ((2S, 5R)-5-aminotetrahydro-2H-pyran-2-yl)methanol (**21t**) to obtain a desired product. MS m/z: 479.1 (M+H)⁺.

### Example 67: (1r, 4r)-4-((5-(2-chloro-4-phenoxybenzoyl)-7H-pyrrolo[2,3-c]pyridazin-4-yl)amino)cyclohexane-1-carboxylic acid (I-67)

The compound was prepared following the general Scheme **4** with Intermediate **8s** (Table 1) and methyl (1r, 4r)-4-aminocyclohexane-1-carboxylate (**21a**) to obtain a desired compound. MS m/z: 491.1 (M+H)⁺.

### Example 68: (2-chloro-4-phenoxyphenyl)(4-(((1r, 4r)-4-(hydroxymethyl)cyclohexyl)amino)-7H-pyrrolo[2,3-c]pyridazin-5-yl)methanone (I-68)

The compound was prepared following the general Scheme **4** with Intermediate **8r** (Table 1) and ((1r, 4r)-4-aminocyclohexyl)methanol (**21r**) to obtain a desired product.

MS m/z: 477.2 (M+H)⁺.

### Example 69: (2-chloro-4-phenoxyphenyl)(4-(((3R, 6S)-6-(hydroxymethyl)tetrahydro-2H-pyran-3-yl)amino)-7H-pyrrolo[2,3-c]pyridazin-5-yl)methanone (I-69)

The compound was prepared following the general Scheme **4** with Intermediate **8s** (Table 1) and ((2S, 5R)-5-aminotetrahydro-2H-pyran-2-yl)methanol (**21t**) to obtain a desired product. MS m/z: 479.1 (M+H)⁺.

### Example 70: (1r, 4r)-4-((3-(2-chloro-4-phenoxybenzoyl)-1H-pyrazolo[3,4-d]pyrimidin-4-yl)amino)cyclohexane-1-carboxylic acid (I-70)

The compound was prepared following the general Scheme **4** with Intermediate **8t** (Table 1) and methyl (1r, 4r)-4-aminocyclohexane-1-carboxylate (**21a**), to obtain a desired compound. MS m/z: 492.1 (M+H)⁺.

### Example 71: (2-chloro-4-phenoxyphenyl)(4-(((1r, 4r)-4-(hydroxymethyl)cyclohexyl)amino)-1H-pyrazolo[3,4-d]pyrimidin-3-yl)methanone (I-71)

The compound was prepared following the general Scheme **4** with Intermediate **8t** (Table 1) and ((1r, 4r)-4-aminocyclohexyl)methanol (**21r**) to obtain a desired product.

MS m/z: 478.2 (M+H)⁺.

### Example 72: (2-chloro-4-phenoxyphenyl)(4-(((3R, 6S)-6-(hydroxymethyl)tetrahydro-2H-pyran-3-yl)amino)-1H-pyrazolo[3,4-d]pyrimidin-3-yl)methanone (I-72)

The compound was prepared following the general Scheme **4** with Intermediate **8t** (Table 1) and ((2S, 5R)-5-aminotetrahydro-2H-pyran-2-yl)methanol (**21t**) to obtain a desired product. MS m/z: 480.1 (M+H)⁺.

### Example 73: (4-(((1r, 4r)-4-aminocyclohexyl)amino)-1H-pyrrolo[2,3-b]pyridin-3-yl)(2-chloro-4-phenoxyphenyl)methanone (I-73)

The compound was prepared following general Scheme **4** with Intermediate **8a** (Table 1) and t-butyl ((1r, 4r)-4-aminocyclohexyl)-1-carbamate (**21v**) to obtain a desired compound.

MS m/z: 461.2 (M+H)⁺; ¹H NMR (DMSO-d6, 400 MHz): δ = 8.68 (s, 1H), 8.04 (s, 2H), 7.94 (d, 1H), 7.55-7.53(m, 2H), 7.50-7.46 (m, 2H), 7.27-7.24 (m, 1H), 7.20-7.18 (m, 3H), 7.03-7.00 (m, 1H), 6.45 (d, 1H), 3.49-3.45 (m, 1H), 3.13-3.11 (m, 1H), 2.18-2.15 (m, 2H), 2.04-2.01 (m, 2H), 1.56-1.53 (m, 2H), 1.45-1.42 (m, 2H).

### Example 74: (4-(((1s, 4s)-4-aminocyclohexyl)amino)-1H-pyrrolo[2,3-b]pyridin-3-yl)(2-chloro-4-phenoxyphenyl)methanone (I-74)

The compound was prepared following general Scheme **4** with Intermediate **8a** (Table 1) and t-butyl ((1s, 4s)-4-aminocyclohexyl)carbamate (**21w**) to obtain a desired product.

MS m/z: 461.2 (M+H)⁺; ¹H NMR (DMSO-d6, 400 MHz): δ = 8.72 (s, 1H), 8.00 (s, 2H), 7.93 (d, 1H), 7.56-7.54(m, 2H), 7.50-7.46 (m, 2H), 7.27-7.24 (m, 1H), 7.19-7.17 (m, 3H), 7.04-7.01 (m, 1H), 6.32 (d, 1H), 3.75-3.71 (m, 1H), 3.17-3.15 (m, 1H), 1.88-1.78 (m, 4H), 1.76-1.72 (m, 4H). **Example 75:** (S)-(2-chloro-4-phenoxyphenyl)(4-(pyrrolidin-3-ylamino)-1H-pyrrolo[2,3-b]pyridin-3-yl)methanone(**I-75**)

The compound was prepared following general Scheme **4** with Intermediate **8a** (Table 1) and t-butyl (S)-3-aminopyrrolidin-1-carboxylate (**21x**) to obtain a desired product.

MS m/z: 433.1 (M+H)⁺.

### Example 76: (R)-(2-chloro-4-phenoxyphenyl)(4-(pyrrolidin-3-ylamino)-1Hpyrrolo[2,3-b]pyridin-3-yl)methanone (I-76)

The compound was prepared following general Scheme **4** with Intermediate **8a** (Table 1) and t-butyl (R)-3-aminopyrrolidin-1-carboxylate (**21y**) to obtain a desired product.

MS m/z: 33.1 (M+H)⁺; ¹H NMR (DMSO-d6, 400 MHz): δ = 8.89 (d, 1H), 8.11 (d, 1H), 7.71 (s, 1H), 7.64-7.56 (m, 3H), 7.37-7.33 (m, 1H), 7.29-7.28 (m, 3H), 7.14-7.11 (m, 1H), 6.49 (d, 1H), 4.47-4.46 (m, 1H), 3.72-3.70 (m, 1H), 3.16-3.14 (m, 4H), 2.54-2.50 (m, 1H), 2.10-2.09 (m, 1H). **Example 77:** (R)-(2-chloro-4-phenoxyphenyl)(4-((1-(methanesulfonyl)piperidin-3-yl)amino)-1H-pyrrolo[2,3-b]pyridin-3-yl)methanone (**I-77**)

The compound was prepared following general Scheme **8** with Intermediate **26** and methylsulfonyl chloride (**29b**) to obtain a desired product;

MS m/z: 554 (M+H)⁺.

### Example 78: (4-(((1r, 3r)-3-aminocyclobutyl)amino)-1H-pyrrolo[2,3-b]pyridin-3-yl)(2-chloro-4-phenoxyphenyl)methanone (I-78)

The compound was prepared following general Scheme **4** with Intermediate **8a** (Table 1) and t-butyl ((1r, 3r)-3-aminocyclobutyl)carbamate **(21aa)** to obtain a desired product.

MS m/z: 433.2 (M+H)⁺.

### Example 79: (4-(((1s, 3s)-3-aminocyclobutyl)amino)-1H-pyrrolo[2,3-b]pyridin-3-yl)(2-chloro-4-phenoxyphenyl)methanone (I-79)

The compound was prepared following general Scheme **4** with Intermediate **8a** (Table 1) and t-butyl ((1s, 3s)-3-aminocyclobutyl)carbamate **(21bb)** to obtain a desired product.

MS m/z: 433.2 (M+H)⁺.

### Example 80: (4-(((1R, 3R)-3-aminocyclopentyl)amino)-1H-pyrrolo[2,3-b]pyridin-3-yl)(2-chloro-4-phenoxyphenyl)methanone (I-80)

The compound was prepared following general Scheme **4** with Intermediate **8a** (Table 1) and t-butyl ((1R, 3R)-3-aminocyclopentyl)carbamate **(21cc)** to obtain a desired product.

MS m/z: 447.2 (M+H)⁺.

### Example 81: (4-((1S, 3R)-3-aminocyclopentyl)amino)-1H-pyrrolo[2,3-b]pyridin-3-yl)(2-chloro-4-phenoxyphenyl)methanone (I-81)

The compound was prepared following general Scheme **4** with Intermediate **8a** (Table 1) and t-butyl ((1R, 3S)-3-aminocyclopentyl)carbamate **(21dd)** to obtain a desired product.

MS m/z: 447.2 (M+H)⁺.

### Example 82: (4-(((1R, 3R)-3-aminocyclohexyl)amino)-1H-pyrrolo[2,3-b]pyridin-3-yl)(2-chloro-4-phenoxyphenyl)methanone (I-82)

The compound was prepared following general Scheme **4** with Intermediate **8a** (Table 1) and t-butyl ((1R, 3R)-3-aminocyclohexyl)carbamate **(21ee)** to obtain a desired product;

MS m/z: 461.2 (M+H)⁺.

### Example 83: (4-(((1S, 3R)-3-aminocyclohexyl)amino)-1H-pyrrolo[2,3-b]pyridin-3-yl)(2-chloro-4-phenoxyphenyl)methanone (I-83)

The compound was prepared following general Scheme **4** with Intermediate **8a** (Table 1) and t-butyl ((1R, 3S)-3-aminocyclohexyl)carbamate **(21ff)** to obtain a desired product.

MS m/z: 461.2 (M+H)⁺.

### Example 84: N-((1r, 4r)-4-((3-(2-chloro-4-phenoxybenzoyl)-1H-pyrrolo[2,3-b]pyridin-4-yl)amino)cyclohexyl)methanesulfonamide (I-84)

The compound was prepared following general Scheme **8** with Product **I-73** and methylsulfonyl chloride (**27b**) to obtain a desired product.

MS m/z: 539.2 (M+H)⁺.

### Example 85: N-((1s, 4s)-4-((3-(2-chloro-4-phenoxybenzoyl)-1H-pyrrolo[2,3-b]pyridin-4-yl)amino)cyclohexyl)methane sulfonamide (I-85)

The compound was prepared following general Scheme **8** with Product **I-74** and methylsulfonyl chloride (**27b**) to obtain a desired product.

MS m/z: 539.2 (M+H)⁺; ¹H NMR (DMSO-d6, 400 MHz): δ = 12.5 (s, 1H), 8.85-8.83 (d, 1H), 7.94-7.92 (d, 1H), 7.57-7.55 (m, 2H), 7.50-7.46(m, 2H), 7.27-7.23 (m, 1H), 7.19-7.14 (m, 4H), 7.04-7.01 (m, 1H), 6.38-6.36 (d, 1H), 3.69 (s, 1H), 3.36 (s, 1H), 2.918 (s, 3H), 1.79-1.75 (m, 8H). **Example 86:** N-((1r, 4r)-4-((3-(2-chloro-4-phenoxybenzoyl)-1H-pyrrolo[2,3-b]pyridin-4-yl)amino)cyclohexyl)acetamide (**I-86**)

The compound was prepared following general Scheme **8** with Product **I-73** and acetyl chloride (**27c**) to obtain a desired product.

MS m/z: 503.2 (M+H)⁺.

### Example 87: N-((1s, 4s)-4-((3-(2-chloro-4-phenoxybenzoyl)-1H-pyrrolo[2,3-b]pyridin-4-yl)amino)cyclohexyl)acetamide (I-87)

The compound was prepared following general Scheme **8** with Product **I-74** and acetyl chloride (**27c**) to obtain a desired product.

MS m/z: 503.2 (M+H)⁺; ¹H NMR (DMSO-d6, 400 MHz): δ = 9.52 (s, 1H), 7.79 (d, 1H), 7.42-7.37 (m, 4H), 7.27-7.23(m, 1H), 7.10-7.06 (m, 3H), 6.96-6.93 (m, 1H), 6.27 (d, 1H), 5.66 (d 1H), 5.30 (s, 1H), 3.96-3.94 (m, 1H), 3.81-3.80 (m, 1H), 1.962 (s, 3H), 1.95-1.86 (m, 2H), 1.87-1.80 (m, 4H), 1.76-1.71 (m, 2H).

### Example 88: (2-chloro-4-phenoxyphenyl)(4-(((1r, 4r)-4-(methylamino)cyclohexyl)amino)-1H-pyrrolo[2,3-b]pyridin-3-yl)methanone (I-88)

The compound was prepared following general Scheme **4** with Intermediate **8a** (Table 1) and t-butyl ((1r, 4r)-4-aminocyclohexyl)(methyl)carbamate (**21gg**) to obtain a desired product. MS m/z: 475.2 (M+H)⁺.

### Example 89: (2-chloro-4-phenoxyphenyl)(4-((1s, 4s)-4-(methylamino)cyclohexyl)amino)-1H-pyrrolo[2,3-b]pyridin-3-yl)methanone (I-89)

The compound was prepared following general Scheme **4** with Intermediate **8a** (Table 1) and t-butyl ((1s, 4s)-4-aminocyclohexyl)(methyl)carbamate **(**21hh**)** to obtain a desired product.

MS m/z: 475.2 (M+H)⁺; ¹H NMR (DMSO-d6, 400 MHz): δ = 12.5 (s, br, 1H), 8.73-8.71 (M, 1H), 8.51 (s, br, 1H), 7.94-7.92 (d, 1H), 7.56-7.53 (m, 2H), 7.50-7.46(m, 2H), 7.27-7.23 (m, 1H), 7.19-7.17 (m, 3H), 7.04-7.01 (m, 1H), 6.33-6.32 (d, 1H), 3.78 (s, 1H), 3.11 (s, 1H), 2.55 (s, 3H), 1.93-1.91 (m, 4H), 1.77-1.75 (m, 4H).

### Example 90: Biological activity study:

Inhibitory activity test on **BTK** kinase and **BTK C481S** kinase:
The inhibitory activities of some compounds of the present invention were determined using the protocols listed below.

The activities of **BTK** kinase and **BTK C481S** kinase were determined by electrophoretic mobility shift assay.

A 1X kinase buffer was prepared. The compound was serially 3-fold diluted with dimethyl sulfoxide over gradients, with a total of 10 concentrations (100000, 33333.33, 11111.11, 3703.70, 1234.57, 411.52, 137.17, 45.72, 15.24, and 5.08 nM), to prepare a 100X final solution. The final starting concentration of the test compound is 1 M (1000 nM).µ

250 nL of the compound of 100X final concentration was transferred to a 384-well microplate by a dispenser. The compound was diluted with the 1X kinase buffer to prepare a kinase solution of 2.5X final concentration. 10 µL of the kinase solution of 2.5X final concentration was transferred to a 384-well microplate. 10 µL of 1X kinase buffer was added to the negative control well. The enzyme and compound were cultured at room temperature for 10 min. A mixed solution of ATP and substrate of 5/3X final concentration was prepared with the 1X kinase buffer. 15 µL of mixed solution of ATP and substrate of 5/3X final concentration was added into a 384-well plate and reacted at room temperature. 30 µL quenching buffer was added to terminate the reaction.

The conversion rate was read on Caliper EZ Reader!!.

### Data analysis

(1) %Inh = (maximum signal-composite signal)/(maximum signal-minimum signal) x 100.
(2) In the absence of the compound, the maximum signal is obtained.
(3) In the absence of the enzyme, the minimum signal was obtained.

Table 1 below lists the IC₅₀ values of the compounds for each enzyme.

**Table 1. In-vitro inhibitory test results of various compounds on kinases BTK and BTK C481S (unit: nM)**

| **Compounds** | **BTK** | **BTK C481S** | **Compounds** | **BTK** | **BTK C481S** |
|---|---|---|---|---|---|
| **I-5** | 9.0 | 1.9 | **I-32** | 11 | 6.2 |
| **I-6** | 2.7 | 1.2 | **I-33** | 80 | 32 |
| **I-8** | 7.1 | 24 | **I-35** | 22 | 6.4 |
| **I-11** | 6.2 | 3.1 | **I-37** | 141 | 14 |
| **I-22** | 9.5 | 7.0 | **I-38** | 242 | 27 |
| **I-23** | 48 | 32 | **I-40** | 549 | 178 |
| **I-24** | 52 | 13 | **I-41** | 245 | 115 |
| **I-28** | 110 | 47 | **I-73** | 16 | 5.7 |
| **I-30** | 95 | 11 | **I-74** | 2.8 | 1.7 |
| **I-31** | 28 | 11 | **I-77** | 172 | 32 |
| **ARQ531** | 9.0 | 2.5 | | | |

### Cell assay:

Plating: The cells were suspended in serum-free medium and counted with automatic cell counter. According to the seeding density, the cell suspension was diluted to the required density. 95 µL cell (2000 cells/well) was added to each well, and incubated at 5% CO₂ and 37°C.

Preparation of compound solution: The compound was prepared into a diluted solution of 1000X final concentration with dimethyl sulfoxide. The test concentrations of the compound were 10000, 3333.33, 1111.11, 370.37, 123.46, 41.15, 13.72, 4.57, 1.52, and 0.51 nM. The compound was diluted with the culture medium to prepare a compound solution of 20X final concentration. 5 uL of the compound solution was added to each well, and the same volume of dimethyl sulfoxide was added to the control well. The cells were incubated at 37°C and 5% CO₂ for 72 hrs.

Detection: The cell plate equilibrated to room temperature, 40 µl of CellTiter-Glo^{®} reagent was added to each well, shaken for 2 min, allowed to stand for 10 min, and detected with SpectraMax Paradigm.

Data analysis:
(1) The IC50 value was calculated using GraphPad Prism 5.
(2) %lnh = (Max signal - Compound signal) / (Max signal - Min signal) x 100.
(3) Max signal was a value of the positive control well in which only DMSO of the same volume with the compound solution was present.
(4) Min signal was a value of the negative control well in which only the culture medium was present.

The test results are shown in Table 2.

**Table 2. In-vitro inhibitory test results of various compounds on cells TMD8 and Ba/F3-BCR-BTK-C481S (unit: nM)**

| **Compounds** | **TMD8** | **Ba/F3-BCR-BTK-C481S** |
|---|---|---|
| I-74 | 65.90 | 52.01 |
| I-87 | 114.20 | 102.60 |
| I-89 | 97.68 | 68.62 |
| ARQ531 | 111.20 | 447.10 |

While preferred embodiments of the present invention have been described above, the present invention is not limited thereto. It should be appreciated that some improvements and variations can be made by those skilled in the art without departing from the technical principles of the present invention, which are also contemplated to be within the scope of the present invention.

## Claims

1. A compound of Formula I, an enol compound, a pharmaceutically acceptable salt, a solvate and a composition thereof, wherein R, G, W¹, W², W³, X, and Y are as defined herein, 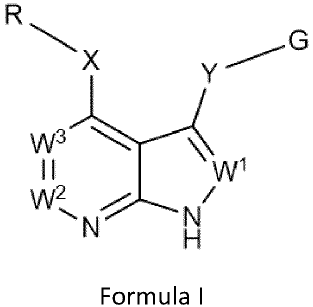 wherein
R is independently selected from: a substituted or unsubstituted alkyl, an alkenyl group, an alcohol in which an alkyl group is unsubstituted or substituted, an amine in which an alkyl group is unsubstituted or substituted, a substituted or unsubstituted four- to seven-membered carbocyclic ring, and a substituted or unsubstituted four- to seven-membered carbocyclic ring having a heteroatom which comprises, but not limited to, N, S, and O;
G is independently selected from a five- or six-membered substituted or unsubstituted aryl group, a substituted or unsubstituted heteroaryl group, a biphenyl, a carbocyclic ring, a saturated or unsaturated carbocyclic ring containing a heteroatom;
W¹, W², and W³ are independently selected from CH or N that satisfy the valence bond theory;
X is independently selected from O, N, and NH;
Y is independently selected from, but is not limited to, the followings: 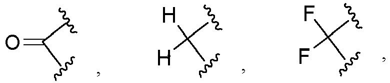
where when Y is absent, there is a chemical bond between the G unit and the heteroaryl ring.

2. A pharmaceutical composition, comprising any compound of general Formula I according to claim 1, or a stereoisomer, a tautomer, a pharmaceutically acceptable salt, a hydrate or a solvate thereof, and a pharmaceutically acceptable carrier.

3. The compound of Formula I according to claim 1, and an enol salt, a pharmaceutically acceptable salt, a stereoisomer, a deuterated derivative, a hydrate or a solvate thereof, wherein the compound is selected from any one of the followings:

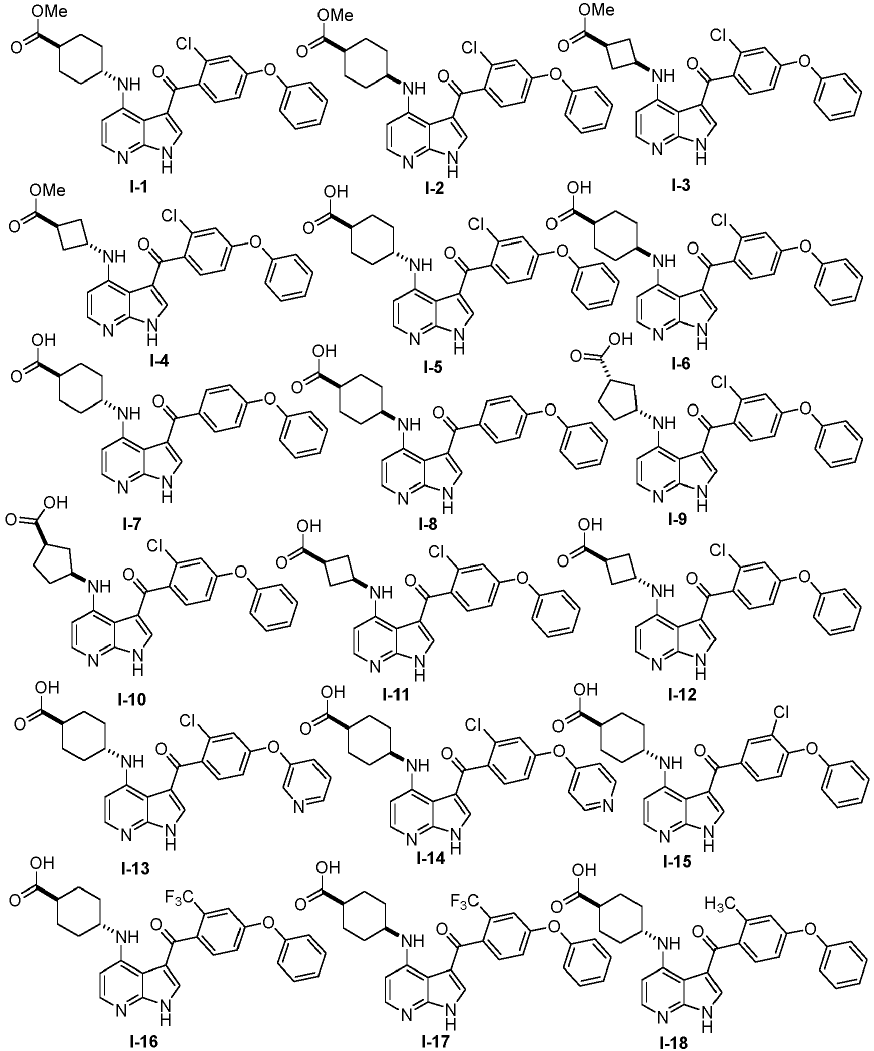

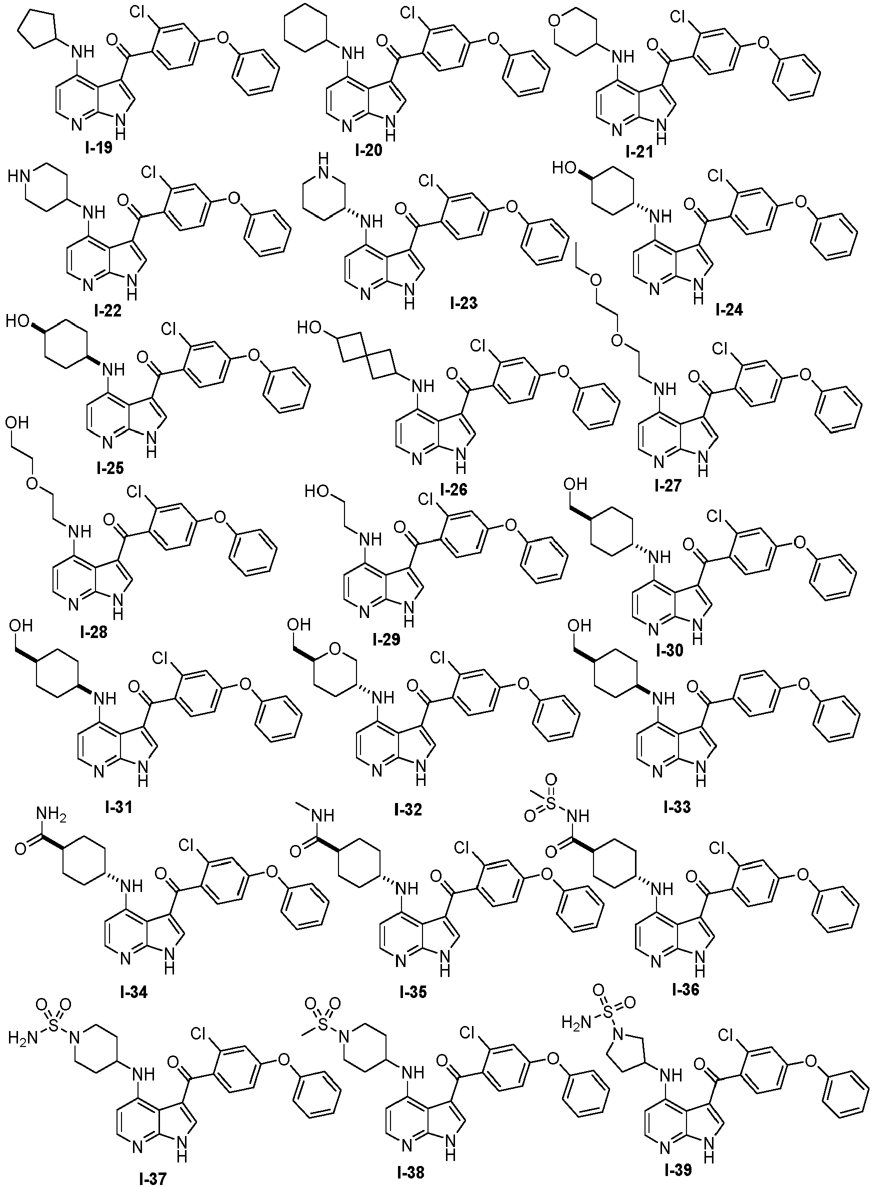

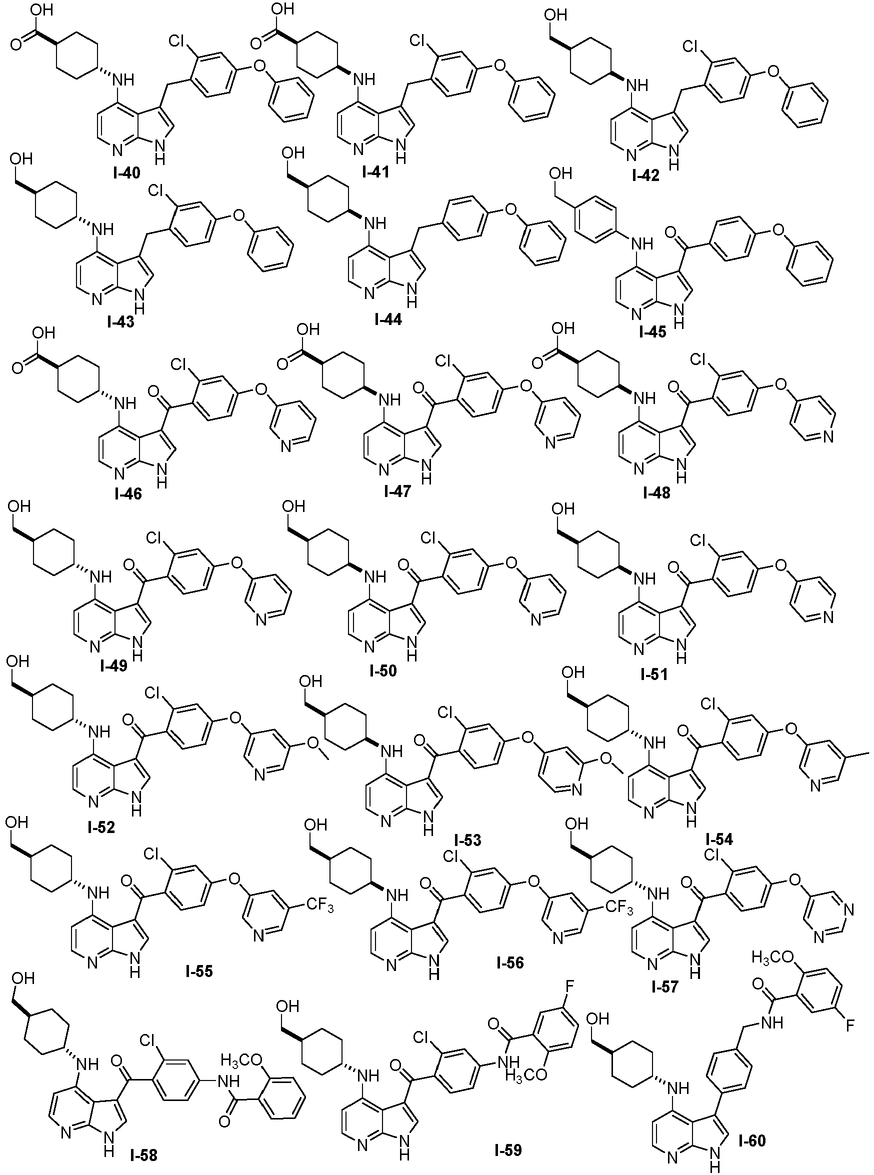

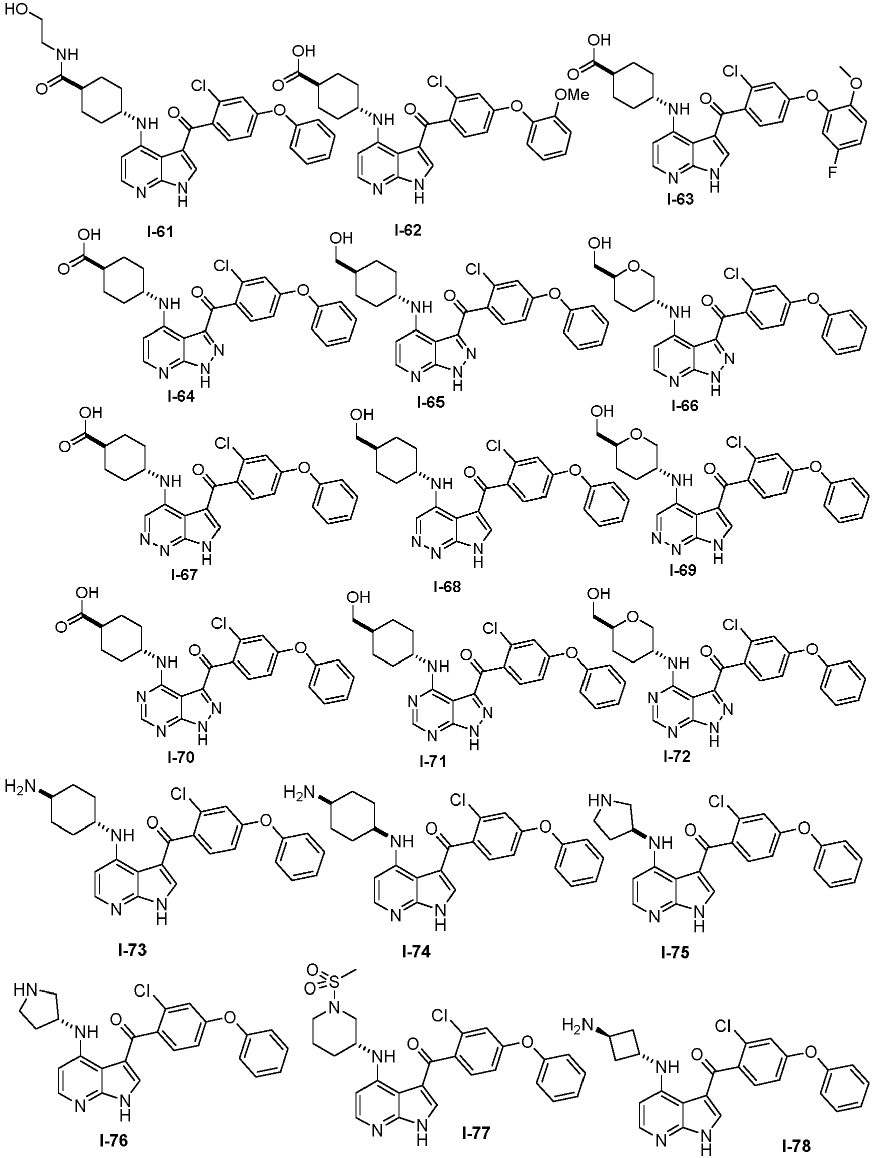

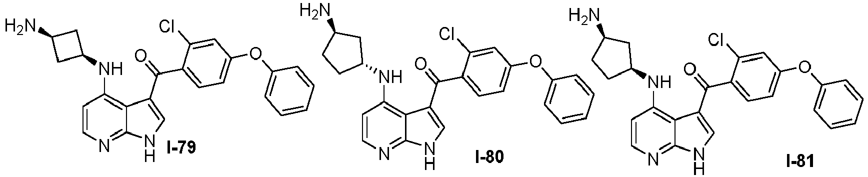

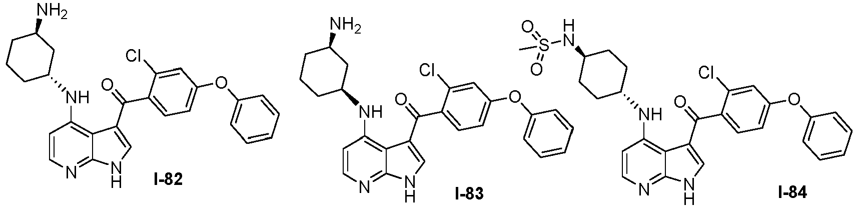

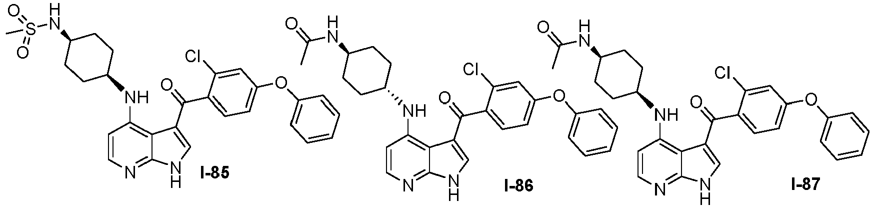

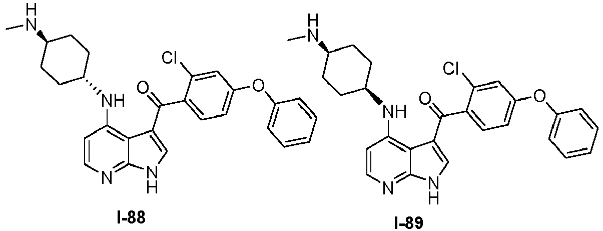

4. A pharmaceutical composition, comprising a compound or an enoate salt, a isomer or a pharmaceutically acceptable salt, a stereoisomer, a deuterated derivative, a hydrate or a solvate thereof, a solvate of any compound according to claims 1 to 3, and a pharmaceutically acceptable carrier or excipient.

5. Use of the compound according to any one of claims 1 to 3 or the pharmaceutical composition according to claim 4, wherein the drug is used alone or in combination with other therapeutic agents.

6. A method for treating a subject suffering from a medically treated condition, by controlling the kinase activity of a Bruton's tyrosine kinase inhibitor or being selective for the BTK (C481S) mutant, or by administering a therapeutically effective amount of the compound according to any one of claims 1 to 4 or the pharmaceutical composition according to claim 5 to the subject.

7. A method for treating a subject that is resistant to cancers related to the activity of Bruton's tyrosine kinase or BTK (C481S) mutant, comprising administering a therapeutically effective amount of the compound according to any one of claims 1 to 5 or the pharmaceutical composition according to claim 6 to the subject.

8. Use of the compound according to any one of claims 1 to 6, or a stereoisomer, a tautomer, or a pharmaceutically acceptable salt thereof, or the hydrate, or the solvate or the pharmaceutical composition according to claim 7, in the preparation of drugs for preventing and/or treating diseases associated with abnormal activity of BTK and BTK mutant (for example, C481S).

9. Use of the drug or composition according to claim 7 or 8 in the treatment of diseases associated with Bruton's tyrosine kinase or BTK(C481S) mutant, autoimmune diseases or inflammatory diseases or cancers, including, but not limited to, B cell-derived malignant tumor (MCL), chronic lymphoblastic leukemia (CLL), acute lymphoblastic leukemia (ALL), non-Hodgkin's lymphoma (NHL), Waldenstrom macrogranulocytia (WM) and multiple myeloma (MM), intravascular large B cell lymphoma, primary exudative lymphoma, Burkitt's lymphoma/leukemia or lymphomatoid granulomatosis.

10. The use according to any one of claims 5 to 9, wherein the drug is administered orally, parenterally, intravenously or transdermally.
